# EUROPEAN PATENT APPLICATION

(11) **EP 4 115 900 A1**
(43) Date of publication of application: **11.01.2023**
(21) Application number: 21305931.4
(22) Date of filing: 05.07.2021
(51) Int. Cl.: A61K 39/02, A61K 39/085, A61K 39/12, A61P 31/18

(54) **NOVEL ANTIGENS AND VACCINES**

(71) Applicant: Diaccurate, 75014 Paris (FR)
(72) Inventor: The designation of the inventor has not yet been filed
(74) Representative: Cabinet Becker et Associés

(57) **Abstract**

The present invention relates to novel polypeptides, nucleic acids, vaccine compositions and the uses thereof. The invention particularly relates to vaccines comprising an antigen (polypeptide, peptide, cell, nucleic acid, vector) having one or more modified 3S motifs. Such vaccines provide improved protecting effect and increased co-stimulation of CD8 T cells and B -cells by CD4 T cells.

## Description

The present invention relates to novel polypeptides, nucleic acids, vaccine compositions and the uses thereof. The invention particularly relates to vaccines comprising an antigen (polypeptide, peptide, cell, nucleic acid, vector) having one or more modified 3S motifs. Such vaccines provide improved protecting effect and increased co-stimulation of CD8 T cells and B -cells by CD4 T cells.

### Background of the invention

The HIV pandemic remains a major global health challenge. There is broad scientific consensus that the most effective approach to control the HIV epidemic is to develop a preventive AIDS vaccine. However, despite over thirty years of HIV research and numerous vaccine trials, there is no vaccine against HIV on the market (Ng'uni T et al. Major Scientific Hurdles in HIV Vaccine Development: Historical Perspective and Future Directions. Frontiers in Immunology (2020) doi.org/10.3389/fimmu.2020.590780)

Initially, scientists believed that neutralizing antibodies would be adequate to protect against HIV infection and many of the HIV vaccines in this category were designed to primarily target the envelope glycoproteins, gp120 or gp160 (Esparza J. A brief history of the global effort to develop a preventive HIV vaccine. Vaccine (2013) 31:3502-18. doi: 10.1016/j.vaccine.2013.05.018). The first experimental immunization of humans against HIV/AIDS was done in Zaire by Zagury and colleagues in 1986 (Zagury D, Leonard R, Fouchard M, Reveil B, Bernard J, Ittele D, et al. Immunization against AIDS in humans. Nature (1987) 326:249-50. doi: 10.1038/326249a0). Following this pioneering in human experimental HIV/AIDS vaccine trial, over 250 clinical trials have been carried out, with the majority being earlyphase trials (phase 1 or 2) (Wang HB, Mo QH, Yang Z. HIV vaccine research: the challenge and the way forward. J Immunol Res (2015) 2015:503978. doi: 10.1155/2015/503978).

Multiple failures in antibody-based vaccines such as the VaxGen gp120 trials prompted the HIV vaccine field to begin pursuing T cell-based vaccines. The first large T cell-based vaccine trial (3000 participants), the STEP trial also called HVTN 502 trial (ClinicalTrials.gov Identifier: NCT00095576), was a phase 2b multicenter, double-blind, randomized, placebo-controlled "test-of-concept" study that tested the efficacy of the MRKAd5 HIV-1gag/pol/nef vaccine. Another trial, the MRKAd5 HIV-1gag/pol/nef vaccine was also tested in a phase 2b trial (the Phambili/HVTN 503 trial; ClinicalTrials.gov Identifier: NCT00413725) in 801 South African adults. Unfortunately, vaccination and enrolment into both the STEP and Phambili trials were terminated in September 2007 following preliminary assessment that demonstrated no efficacy (Esparza J. A brief history of the global effort to develop a preventive HIV vaccine. Vaccine (2013) 31:3502-18. doi: 10.1016/j.vaccine.2013.05.018; Cohen J. AIDS research. Promising AIDS vaccine's failure leaves field reeling. Science (2007) 318:28-9. doi: 10.1126/science.318.5847.28). Furthermore, multivariate analysis of baseline risk factors revealed that the vaccination resulted in increased risk of HIV infection in some volunteers.

To date, only the RV144 trial (ClinicalTrials.gov Identifier: NCT00223080) has shown modest efficacy of 31.2% 42 months after the final vaccination (Ng'uni T et al. Major Scientific Hurdles in HIV Vaccine Development: Historical Perspective and Future Directions. Frontiers in Immunology (2020) doi.org/10.3389/fimmu.2020.590780).

Although considerable progress has been made in understanding the structural and molecular biology of the HIV virus, there is still a need for new and efficient vaccinal approaches against HIV.

It has been previously documented by the inventors that PLA2G1B is involved in the inactivation of CD4 T cells in HIV infected patients (see WO2015/097140). It was thus proposed by the inventors that PLA2G1B modulators are effective for treating diseases in mammal, e.g., disorders associated with an immune deficiency.

Continuing their research, the inventors have now found that the effect of PLA2G1B can be mediated and/or amplified by a cofactor present in diseased subjects, and that such cofactor acts through a gC1q receptor at the surface of T cells (see WO2019/166412). The inventors have also shown that pathogens produce or activate a cofactor which binds to gC1qR, leading to a sensitization of CD4 T cells to inactivation by very low doses of PLA2G1B. In patients infected with such pathogens, CD4 T cells become sensitive to inactivation by physiological amounts of PLA2G1B, while in non-infected subjects, CD4 T cells remain resistant to inactivation by such physiological concentration of PLA2G1B. The inventors have identified such gC1qR-binding cofactors from various pathogens, including viruses or bacteria, such as HIV, HCV or S. aureus. The inventors have also verified that said cofactors could increase PLA2G1B enzymatic activity on CD4 T cells. The HIV-derived cofactor is present in HIV-infected viremic patients (VP) plasmas and sensitizes CD4 T cells to inactivation by PLA2G1B of response to IL-7 stimulation. The cofactor activity of HIV VP plasmas was due to the conserved 3S domain of the gp41 HIV-1 envelope protein as immunodepletion of HIV VP plasmas with anti-gp41 polyclonal antibodies or anti-3S monoclonal antibody almost abrogate the PLA2G1B-dependent inhibition of CD4 T cells response. This suggests that blocking of such cofactors *in vivo* could restore or maintain resistance of CD4 T cells to inactivation by PLA2G1B. Applicant thus identified a novel general mechanism by which many pathogens induce diseases or pathological conditions in mammals, i.e., by inducing a sensitization of CD4 T cells to inactivation by PLA2G1B. Such previous findings allowed applicant to provide novel therapeutic approaches based on an inhibition of the cofactor effect.

The present invention stems from the finding that the 3S motif(s) contained in various antigens used for vaccination may actually affect the efficacy of such vaccines by inducing a PLA2G1B cofactor effect. The inventors have hypothesized that the presence of such 3S motif(s) may, in fact, be responsible for the failure to develop potent and efficient anti-HIV vaccines due to inhibition of CD4 T cells response by PLA2G1B. As CD4 T cells are critical to provide the co-stimulation signal required for a potent stimulation of B-cells and CD8 T cells, impairment of CD4 T cells due to the activation of PLA2G1B by 3S motifs present in HIV antigens could explain the failure of vaccination against HIV antigens.

The invention now shows that, by modifying antigens in order to alter or delete one or several 3S motif(s) present in their sequence, improved vaccine efficacy can be obtained. In particular, such modified antigens retain potent immunogenicity while showing reduced PLA2G1B cofactor effect. Such antigens thus cause substantially less, or no, CD4 T cell anergy and thus cause much stronger immune protection.

### Summary of the invention

It is an object of the present invention to provide new modified polypeptides, wherein said polypeptides comprise at least one modified 3S motif, and wherein said modified polypeptide have reduced PLA2G1B cofactor effect on CD4 T cells (as compared to corresponding polypeptides without said modified 3S motif).

It is an object of the present invention to provide an antigen suitable for use in a vaccine, wherein said antigen comprises at least one modified 3S motif, and wherein said antigen has reduced PLA2G1B cofactor effect on CD4 T cells as compared to the corresponding antigen without said modified 3S motif.

The polypeptides or antigens of the invention may be of various origin, such as viral, bacterial, cellular, etc.

A particular object of the invention relates to a new modified HIV polypeptide, wherein said polypeptide comprises a modified 3S motif, and wherein said modified polypeptide has reduced PLA2G1B cofactor effect on CD4 T cells as compared to a HIV polypeptide without said modified 3S motif.

The invention also relates to a nucleic acid molecule encoding a polypeptide or antigen as defined above.

The invention also relates to a vector comprising a nucleic acid as defined above.

The invention further relates to a host cell containing a nucleic acid or vector as defined above.

Another object of the invention relates to a modified polypeptide, nucleic acid, vector or cell as described herein, for use as a vaccine.

Another object of the invention relates to a modified polypeptide, nucleic acid, vector or cell as described herein, for use in a method of vaccinating a mammal against HIV.

Another object of the invention relates to a method for vaccinating a mammal, such as a human, comprising administering to the mammal a modified polypeptide, nucleic acid, vector or cell as described herein.

Another object of the invention relates to the use of a modified polypeptide, nucleic acid, vector or cell as described herein, for the manufacture of a medicament for treating a mammal.

Another object of the invention relates to a vaccine comprising a modified polypeptide, nucleic acid, vector or cell according to the invention.

Another object of the invention relates to a composition comprising a modified polypeptide, nucleic acid, vector or cell as described herein.

A further object of the invention relates to a method for preparing a vaccine comprising an antigen, the method comprising (i) modifying a 3S motif present in the antigen, and (ii) formulating the antigen as a vaccine.

A further object of the invention relates to a method for preparing an antigen, comprising (i) selecting an antigen of interest, and (ii) modifying a 3S motif present in the antigen so as to reduce a PLA2G1B cofactor effect of the antigen.

The invention may be used in any mammal, particularly in human subjects. It is suitable to treat diseases wherein a stimulation or restoration of CD4 T cells is beneficial, such as diseases caused by infectious pathogens (e.g., virus or bacteria) or by undesirable cell proliferation (e.g., cancers).

### Legends to the figures

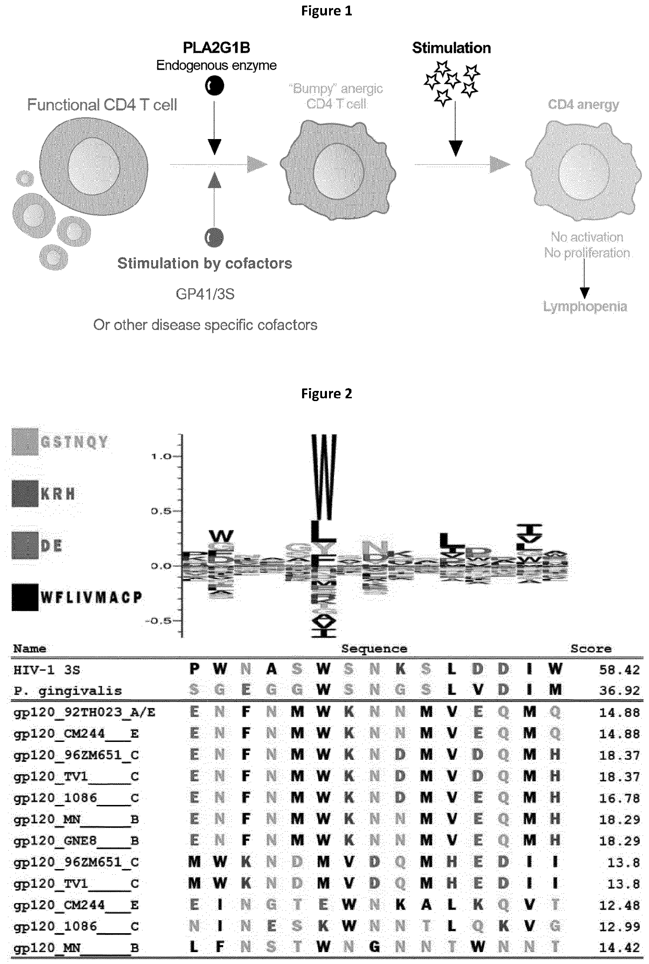

**Figure 1****.** Model of regulation of PLA2G1B activity by disease specific cofactors comprising 3S motif(s): PLA2G1B and PLA2G1B cofactor comprising 3S motif(s) act together on functional CD4 cells leading to CD4 anergy.
**Figure 2****.** Identification of 3S-like immunosuppressive sequences in seven gp120 proteins used in vaccine trials. Position-specific scoring matrix (PSSM) was used to scan several gp120 full-length proteins and allowed to predict 3S-like peptides that could bind to gClqR and increase enzymatic PLA2G1B activity to inhibit CD4 T cells response (presented at the top of the figure). As reference peptide sequences, HIV-1 3S from MN strain and P. gingivalis 3S-like are shown with PSSM value as these two peptides increase PLA2G1B activity, and the critical role of gClqR for the effect has been demonstrated for 3S (see patent WO2019/166413). Peptides with PSSM value >10 are considered as good PLA2G1B cofactor candidates.

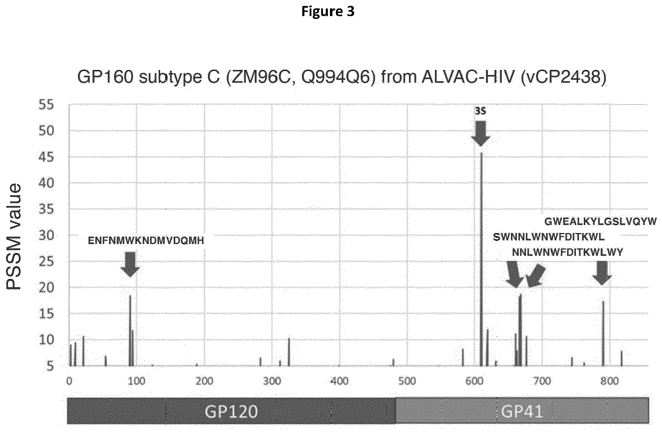

**Figure 3****.** Positions of 3S-like and 3S in the gp160 protein from ALVAC-HIV (vCP2438) vaccine: position-specific scoring matrix (PSSM) values to predict 3S-like peptides that could be PLA2G1B cofactors are indicated for all 15 amino acids peptides in gp160 from 96ZM651 strain (accession number Q994Q6). Amino acids sequence of the 3S and the four 3S-like peptides in gp160 are presented with their relative position of the first amino acid in the gp120 and gp41 subunits of the protein.

### Detailed description

The present invention provides novel polypeptides, vaccine compositions and the uses thereof. In particular, the invention relates to a new modified polypeptide, wherein said polypeptide comprises a modified 3S motif, and wherein said modified polypeptide has reduced PLA2G1B cofactor effect on CD4 T cell as compared to a polypeptide without said modified 3S motif.

### Definitions

As used herein, the term "PLA2G1B" designates group IB pancreatic phospholipase A2. PLA2G1B has been identified and cloned from various mammalian species. The human PLA2G1B protein is disclosed, for instance, in Lambeau and Gelb (2008). The sequence is available on Genbank No. NP_000919. The amino acid sequence of an exemplary human PLA2G1B is shown below (SEQ ID NO: 1):

MKLLVLAVLL TVAAADSGIS PRAVWQFRKM IKCVIPGSDP FLEYNNYGCY CGLGGSGTPV DELDKCCQTH

DNCYDQAKKL DSCKFLLDNP YTHTYSYSCS GSAITCSSKN KECEAFICNC DRNAAICFSK APYNKAHKNL

DTKKYCQS

Amino acids 1 to 15 of SEQ ID NO: 1 (underlined) are a signal sequence, and amino acids 16 to 22 of SEQ ID NO: 1 (in bold) are a propeptide sequence.

Within the context of the invention, the term "PLA2G1B" designates preferably human PLA2G1B.

The human PLA2G1B protein may be present under two distinct forms: a pro-form (pro-sPLA2G1B), which is activated by proteolytic cleavage of a pro-peptide, leading to the mature secreted form (sPLA2G1B). The term PLA2G1B includes any form of the protein, such as the pro-form and/or the mature form. Typically, the mature secreted form comprises the sequence of amino acid residues 23-148 of SEQ ID NO: 1, or any natural variants thereof.

Natural variants of a protein include variants resulting e.g., from polymorphism or splicing. Natural variants may also include any protein comprising the sequence of SEQ ID NO: 1, or the sequence of amino acid residues 23-148 of SEQ ID NO: 1, with one or more amino acid substitution(s), addition(s) and/or deletion(s) of one or several (typically 1, 2 or 3) amino acid residues. Variants include naturallyoccurring variants having e.g., at least 90% amino acid sequence identity to SEQ ID NO: 1. Particular variants contain no more than 10 amino acid substitution(s), addition(s), and/or deletion(s) of one or several (typically 1, 2 or 3) amino acid residues as compared to SEQ ID NO: 1. Typical naturallyoccurring variants retain a biological activity of PLA2G1B. In this regard, in some embodiments, PLA2G1B has at least one activity selected from induction of formation of membrane microdomains (MMD) in CD4 T cells from healthy subjects, or rendering CD4 T cells of healthy subjects refractory to interleukin signaling, such as refractory to IL-2 signaling or refractory to IL-7 signaling or refractory to IL-4 signaling. In some embodiments rendering CD4 T cells of healthy subjects refractory to interleukin-7 signaling comprises a reduction of STAT5A and/or B phosphorylation in said cells by at least about 10%, at least about 20%, at least about 30%, or at least about 40%. In some embodiments rendering CD4 T cells of healthy subjects refractory to interleukin-7 signaling comprises reducing the rate of nuclear translocation of phospho-STAT5A and/or phospho-STAT5B by at least about 20%, at least about 30%, at least about 40%, or at least about 50%.

The term "sequence identity" as applied to nucleic acid or protein sequences, refers to the quantification (usually percentage) of nucleotide or amino acid residue matches between at least two sequences aligned using a standardized algorithm such as Smith-Waterman alignment (Smith and Waterman (1981) J Mol Biol 147:195-197), CLUSTALW (Thompson et al. (1994) Nucleic Acids Res 22:4673-4680), or BLAST2 (Altschul et al. (1997) Nucleic Acids Res 25:3389-3402). BLAST2 may be used in a standardized and reproducible way to insert gaps in one of the sequences in order to optimize alignment and to achieve a more meaningful comparison between them.

The term "inactivation" indicates, in relation to CD4 T cells, that such cells lose at least part of their ability to contribute to the development of an effective immune response. Inactivation may be partial or complete, transient or permanent. Inactivation designates preferably reducing by at least 10%, 20%, 30%, 40%, 50%, 60%, 70%, 80% or more a function of CD4 T cells, particularly pSTAT5 nuclear translocation and/or CD4 T cell's immunostimulatory activity. Typically, inactive CD4 T cells have no effective pSTAT5 nuclear translocation. In a particular embodiment, an inactive CD4 T cell is an anergic CD4 T cell.

The term "resistance" (or "insensitivity") of CD4 T cells to inactivation by PLA2G1B indicates, within the context of this invention, that CD4 T cells are essentially not inactivated *in vitro* when incubated in the presence of 5nM of PLA2G1B. Resistance indicates, for instance, that CD4 T cells retain an active nuclear translocation of pSTAT5 when incubated in vitro in the presence of 5nM PLA2G1B and interleukin-7. Resistance (or insensitivity) of CD4 T cells to PLA2G1B may also indicate that CD4 T cells incubated *in vitro* with 5nM PLA2G1B remain immunologically functional, e.g., do not become anergic.

### PLA2G1B cofactor effect

The term PLA2G1B "cofactor effect" designates a property of an agent (protein, cell, pathogen, etc.) to render cells, particularly CD4 T cells, sensitive (or more sensitive) to inactivation by PLA2G1B. As a result, in the presence of such a cofactor and physiological amounts of PLA2G1B, cells, such as CD4 T cells, become inactive (e.g., anergic) .

The inventors have discovered that many different types of pathogens act as (or produce or activate) a cofactor of PLA2G1B that, in combination with PLA2G1B, leads to CD4 T cell inactivation. The inventors have further discovered that the cofactor effect is mediated by one or more 3S-like motifs contained in the sequence of the cofactor, which allows binding to gClqR at the surface of T cells (WO 2019/166412).

The invention now provides novel polypeptides (such as antigens) which are obtained by modifying one or more 3S motifs, thus providing suitable antigens with reduced or no PLA2G1B cofactor effect.

The present invention demonstrates that modifying polypeptides that have a PLA2G1B cofactor effect, by modifying at least a 3S-like motif, allows to obtain modified antigens with reduced PLA2G1B cofactor effect and which retain potent immunogenicity.

In a preferred embodiment, the PLA2G1B cofactor effect is reduced by at least 20%, more preferably at least 30%, at least 40%, at least 50%, at least 60%, at least 70%, or even more.

In a particular embodiment, the modified polypeptide has reduced immunosuppressive activity on CD4 T cells in comparison with a polypeptide without said modification.

In another preferred embodiment, the modified polypeptide binds gClqR on CD4 T cells with less affinity than a polypeptide without said modification.

In another particular embodiment, the modified polypeptide of the invention is a modified ligand of gC1qR.

The cofactor effect may thus be measured, e.g., in vitro (as disclosed in the present application), by measuring binding to gC1qR, or by measuring CD4 T cell activity (such as by measuring induction of formation of membrane microdomains (MMD) on CD4 T cells, or by measuring responsiveness to interleukin signaling, such as to IL-2 signaling or to IL-7 signaling or to IL-4 signaling. In some embodiments, the cofactor effect is measured by determination of arachidonic acid release by CD4 T cells. For measuring the cofactor effect, the candidate polypeptide is typically incubated with CD4 T cells (preferable human CD4 T cells), optionally in the presence of PLA2G1B. The cofactor effect is then typically compared to that of an unmodified polypeptide, or a reference cofactor effect.

The inventors have identified numerous polypeptides or proteins from multiple pathogens which have one or more 3S-like motifs and can exhibit PLA2G1B cofactor effect, as shown, for example, in below Tables 1, 2 and 3.

Each of said polypeptide may be modified according to the present invention.

In a particular embodiment of the invention, the polypeptide of the invention may be any polypeptide comprising any one of SEQ ID NOs: 2-55 and 72, or any fragment thereof.

The term "fragment", in relation to such polypeptides, designates preferably an immunogenic fragment of a polypeptide, retaining a capacity to induce or stimulate an immune response, preferably against the polypeptide. The immune response may be a cellular or antibody response. The fragment shall contain a modified 3S-motif element of such a polypeptide as well as at least one epitope, thereby having reduced cofactor effect while retaining immunogenic (antigenic) effect. Preferred fragments contain at least 15 consecutive amino acid residues of a polypeptide, typically between 15 and 100, such as between 15 and 90, between 15 and 80, between 20 and 80, between 25 and 75, between 30 and 70, for instance.

### 3S motifs

The inventors have defined a consensus 3S motif, based on various experiments and analyses. The consensus sequence is PWNASWSNKSLDDIW (SEQ ID NO: 72). The central motif of the consensus 3S motif is SWSNKS (SEQ ID NO: 73), which corresponds to amino acids 5 to 10 of SEQ ID NO: 72. Said central motif is a preferred region for modification(s) according to the invention, although every other position may be altered as well, provided it produces a polypeptide with reduced PLA2G1B cofactor effect.

The inventors have more particularly discovered that modifying or deleting a 3S motif present in polypeptides from various pathogens allows to obtain modified polypeptides with reduced PLA2G1B cofactor effect, which can retain potent immunogenicity. Such polypeptides cause substantially less, or no, CD4 T cell anergy, and thus confer efficient immune protection. Such antigens also cause an immune response *in vivo,* for example, an anti-HIV immune response *in vivo,* and may thus be used as vaccines, e.g., anti-HIV vaccines.

The terms '3S motif', '3S-like motif', '3S peptide', '3s peptide motif' are used alternatively in the present application.

Examples of candidate polypeptides and their respective 3S motifs identified by the inventors in numerous proteins are listed in below Tables 1 to 3.

**Table 1: Proteins containing a 3S motif having a PLA2G1B cofactor effect.**

| **PROTEIN NAME** | **ACCESSION NUMBER** | **SPECIES** | **3S motifs (binding to gC1qR)** | **SEQ ID NO** |
|---|---|---|---|---|
| gp41 | AAC31817.1 | HIV | PWNASWSNKSLDDIW | 72 |
| UNKNOWN | WP_077094164.1 | Porphyromonas gingivalis | AWNAIWINRKYEQID | 4 |
| UDP-glucose 4-epimerase | SJM20449.1 | Porphyromonas gingivalis | GIAESWPNSLDDSCA | 5 |
| L-threonine 3-dehydrogenase | WP_013815975.1 | Porphyromonas gingivalis | GIAESWPNSLDDSCA | 6 |
| TonB-dependent receptor | WP_097552718.1 | Porphyromonas gingivalis | SFLKSWFNNSLVDIG | 7 |
| UNKNOWN | WP_097552551.1 | Porphyromonas gingivalis | SGEGGWSNGSLVDIM | 8 |
| DNA polymerase III subunit gamma/tau | SJM20595.1 | Porphyromonas gingivalis | YELDAASNNSVDDIR | 9 |
| Multidrug transporter AcrB | WP_097555277.1 | Porphyromonas gingivalis | AALGKTLVKSLDDIP | 10 |
| Peptide ABC transporter permease | AIF49259.1 | Dyella japonica | PWNASWSDKFYENSL | 11 |
| Peptide ABC transporter substrate binding protein | WP_019421834.1 | Paenibacillus sp. | AIHASWSNTSYEVID | 12 |
| Peptide ABC transporter substrate binding protein | OJX83063.1 | Mesorhizobium sp. | PWNAGWSNARFDELC | 13 |
| MC73_05565 | KGY43685.1 | Proteus mirabilis | PWNAIWSAKNTTVDS | 14 |
| Chitinase | WP_068978097.1 | Aeromonas sp. | PWNASWSAAGVGAHA | 15 |
| TonB-dependent receptor | KZY48959.1 | Pseudoalteromonas sp. | WFNASWKDKSYSTVW | 16 |
| TonB-dependent receptor | WP_036212264.1 | Lysobacter arseniciresistens | PWNASWSVRHISELE | 17 |
| LVIVD repeat protein | EMY15726.1 | Leptospira weilii str. | PWNASWSYVLDSAWS | 18 |
| AMK72_12855 | KPK43807.1 | Planctomycetes bacterium | PWNGSWSNDAWGPGT | 19 |
| Peptide ABC transporter substrate-binding protein | OJX83063.1 | Mesorhizobium sp. | PWNAGWSNARFDELC | 20 |
| UNKNOWN | WP_030088081.1 | Streptomyces baarnensis | PWNAGWSLKSSGKSA | 21 |
| HMPREF0183_2345 | EFG46376.1 | Brevibacterium mcbrellneri | PWNAWWSNRSMIADV | 22 |
| UNKNOWN | WP_048669463 | Vibrio crassostreae | AWNESWSNKSFHNGA | 23 |
| UNKNOWN | WP_070707834.1 | Porphyromonas sp. HMSC077F02 | EFNANWSNKFYLYNQ | 24 |
| FAD-linked oxidoreductase | WP_084705378.1 | Leucobacter chironomi | RWNTSWSNWARTERS | 25 |
| RNA-binding protein 48 | XP_020288140.1 | Pseudomyrmex gracilis | NWNTSWSNTASGSDS | 26 |
| TonB-dependent receptor | WP_083710929.1 | Proteiniphilum saccharofermentans | SINAAWSNQSYGFSR | 27 |
| Peptide ABC transporter | WP_074918487.1 | Terrisporobacter glycolicus | NNNAQWSNKEYDKIV | 28 |
| Type IV secretion protein Rhs | WP_032559173.1 | Bacteroides fragilis | HTCASWCNKSLSDIV | 29 |
| Putative transmembrane rhomboid family protein | CAH09895.1 | Bacteroides fragilis | KDITSWVNKALDAIA | 30 |
| Receptor kinase-like protein Xa21 | XP_020096846.1 | Ananas comosus | GALSSWSNKSLHCCE | 31 |
| Rim ABC transporter | AAC05632.1 | Homo sapiens | EKVANWSIKSLGLTV | 32 |
| Importin beta SMX1 | KTB14942.1 | Candida glabrata | KFIESWSNKSLWLGE | 33 |
| Transporter | WP_049174838.1 | Acinetobacter ursingii | FLLYALSNKSLNDIW | 34 |
| Sugar ABC transporter permease | WP_075333493.1 | Pseudonocardia sp. | PTSVSWSNYEQILVG | 35 |
| ABC transporter substrate binding protein | WP_009846178.1 | Vibrio sp. | DEQKQWRNKSLEQLW | 36 |
| Tetraspanin | NP_001024415.2 | Caenorhabditis elegans | YLGVSWSNKSLLYSY | 37 |
| Nucleotidyltransferase | WP_061886850.1 | Aggregatibacter actinomycetemcomitans | MSKFGLSDKSIEQIH | 38 |
| Phospholipase C, phosphocholine-specific | WP_026813378.1 | Arenibacter certesii | MRYTVESGKSLDDIW | 39 |
| Response regulator of zinc sigma-54-dependent two-component system | WP_087741064.1 | Proteus mirabilis | FELVCASNKSLEQLA | 40 |
| UNKNOWN | WP_018028689.1 | Porphyromonas somerae | DHDKGLETESLEQIW | 41 |
| Peptide ABC transporter permease | WP_065295736.1 | Aggregatibacter aphrophilus | EPKDFRESATLNQIW | 42 |

**Table 2: Selection of proteins with 3S motif(s) associated with human diseases.**

| **PROTEIN** | **3S motifs SEQUENCES** | **SEQ ID NO** | **PATHOLOGY** |
|---|---|---|---|
| gp41 (3S) | PWNASWSNKSLDDIW | 72 | AIDS |
| TonB-dependent receptor (TonBdR Pg) | SFLKSWFNNSLVDIG | 7 | Pancreatic cancer, Chronic periodontal disease Rheumatoid polyarthritis, Alzheimer's Disease |
| Hypothetical protein (HP Pg) | SGEGGWSNGSLVDIM | 8 | |
| MC73_05565 (HP Pm) | PWNAIWSAKNTTVDS | 14 | Urinary tract infections, Osteomyelitis in a HIVpatient |
| LVIVD repeat protein (LVIVD Lw) | PWNASWSYVLDSAWS | 18 | Leptospirosis |
| Peptide ABC transporter (ABC Tg) | NNNAQWSNKEYDKIV | 28 | Wounds infection |
| Type IV secretion protein RhS (Rhs Bf) | HTCASWCNKSLSDIV | 29 | |
| putative transmembrane rhomboid family protein (TM rhomboid Bf) | KDITSWVNKALDAIA | 30 | Peritoneal infections, bacteremia, subcutaneous abscesses or burns |
| Importin beta SMX1 (bSMX1 Cg) | KFIESWSNKSLWLGE | 33 | Cutaneous candidiasis in HIV/AIDS, cancer and organ transplantation |
| Nucleotidyltransferase (Nt trans Aa) | MSKFGLSDKSIEQIH | 38 | Agressive Periodontitis, Bacterial vaginosis, Endocarditis, actinomycosis, Rheumatoid arthritis |
| Hypothetical protein (HP Ps) | DHDKGLETESLEQIW | 41 | Chronic skin, soft tissue and bone infections |
| Peptide ABC transporter permease (ABC Aa) | EPKDFRESATLNQIW | 42 | Endocarditis, brain abscesses, vertebral osteomyelitis and bacteremia |

**Table 3: List of proteins with 3S motif(s).**

| **Pathogen** | **Candidate polypeptide** |
|---|---|
| Virus | HIV gp41 |
| | HIV rev protein |
| | HCV core protein (a.a 26-124) ; SEQ ID NO: 43 |
| | EBV EBNA1 |
| | Adenovirus core protein V |
| | Hantaan virus (HTNV) capsid |
| | HSV Neurovirulence factor ICP34 |
| | Rubella virus protease p150 |
| | Rubella virus capsid protein |
| Bacteria | Staphylococcus aureus protein A ; SEQ ID NO: 44 |
| | Internalin InIB Listeria monocytogenes |
| | Streptococcus pneumoniae hyaluronate lyase |
| | Exosporium of Bacillus cereus |
| Parasite | Plasmodium falciparum |

Other specific sequences of 3S motifs identified by the inventors are 3S-like motifs as shown in any of SEQ ID NO: 106 to 113 of gp120 protein, of any of SEQ ID NO: 114 to 137 of gp 41 protein, of SEQ ID NO: 138 of Hepatitis core protein, of any of SEQ ID NO: 139 to 140 of S. aureus protein A protein, or of any of SEQ ID NO: 141 to 145 of SARS-CoV-2 protein.

Preferably, the modified polypeptides of the invention comprise a modified 3S motif, wherein said 3S motif comprises one or more amino acid modifications selected from a substitution, addition, deletion, and/or chemical alteration, preferably at least 1, 2, 3 or 4 amino acid modifications.

The preferred amino acid modification according to the invention is a substitution of 1, 2, 3, 4 or more amino acid(s) in a 3S motif.

In a more preferred embodiment, the amino acid modification according to the invention is a substitution of 1, 2, 3, 4 or more amino acid(s) in a central motif of a 3S motif (corresponding to amino acids 5 to 10 of the consensus 3S motif of SEQ ID NO: 72), as described above.

In another preferred embodiment, the amino acid modification according to the invention is a substitution of 1, 2, 3, 4 or more amino acid(s), preferably in a central motif of a 3S motif as described above, by alanine residue(s).

The 3S motif may also be partially or fully deleted.

The 3S motif may be mutated at 1, 2, 3 or 4 amino acid residues.

When several 3S motifs are present in a polypeptide, one or more or all of them may be modified, in a same or in a different manner.

It is thus an object of the invention to provide a polypeptide, in particular suitable for vaccinating a mammalian subject, wherein said polypeptide comprises a modified 3S motif, and wherein said modified polypeptide has reduced PLA2G1B cofactor effect on CD4 T cell as compared to a polypeptide without said modified 3S motif.

The present invention relates to any polypeptide, presenting one or more modified 3S-like motif(s) which reduce(s) PLA2G1B cofactor effect on CD4 T cell.

In a particular embodiment, the modified polypeptide according to the invention is a HIV gp140 protein having one or more modified 3S motif(s), or a fragment or variant of such a protein.

In another particular embodiment, the modified polypeptide according to the invention is a HIV gp120 or HIV gp160 protein having one or more modified 3S motif(s), or a fragment or variant of such a protein.

In another particular embodiment, the modified polypeptide according to the invention is a HIV gp41 protein having one or more modified 3S motif(s), or a fragment or variant thereof.

In a further preferred particular, the modified polypeptide according to the invention is a HIV gp41 protein having one or more modified 3S motif(s), wherein the 3S motif(s) has/have a sequence of SEQ ID NO: 72 (PWNASWSNKSLDDIW) comprising 1, 2, 3, 4 or more amino acids (aa) modifications at any position, more preferably at one or more positions located between aa5 to aa10 of SEQ ID NO: 72, i.e. in any of amino acids SWSNKS (SEQ ID NO: 73), more preferably at positions aa6, aa7, aa8 and/or aa10 of SEQ ID NO: 72.

In another preferred embodiment, the modified polypeptide according to the invention is any protein comprising one or more modified 3S motif(s), wherein the 3S motif is the consensus 3S motif PWNASWSNKSLDDIW (SEQ ID NO: 72) comprising 1, 2, 3, 4 or more amino acid(s) modifications at any position, more preferably at positions aa5 to aa10 of SEQ ID NO: 72, more preferably at positions aa6, aa7, aa8 and aa10 of SEQ ID NO: 72.

In another preferred embodiment, the amino acids which are substituted in the modified polypeptides according to the invention are residues homologous to S613, W614, S615, N616 , K617 and S618 of 3S motif in 3S-like sequences of gp120, gp160, gp41 (i.e., SWSNKS of SEQ ID NO: 73), HCV core protein, protein A de Staphylococcus aureus, or Porphyromonas gingivalis, etc. More preferably, the amino acids which are substituted in the modified polypeptides according to the invention are residues homologous to W614, S615, N616 and S618 of 3S motif in 3S-like sequences of gp120, gp160, gp41, HCV core protein, protein A de Staphylococcus aureus, or Porphyromonas gingivalis, etc. Even more preferably, these residues are substituted by 1, 2, 3 or 4 alanine residue(s).

In this regard, in particular embodiments, the modified 3S motifs of gp41 according to the invention are modified 3S motifs having any of SEQ ID NO: 45 to 71.

In other particular embodiments, the modified 3S motifs of gp120 or of gp160 according to the invention, are modified 3S motifs having any of SEQ ID NO: 146 to 151.

In other particular embodiments, the modified 3S motifs of HCV core protein according to the invention, are modified 3S motifs having any of SEQ ID NO: 152 to 153.

In other particular embodiments, the modified 3S motifs of Staphylococcus aureus protein A according to the invention, are modified 3S motifs having any of SEQ ID NO: 154 to 155.

In other particular embodiments, the modified 3S motifs of SARS-CoV-2 protein according to the invention, are modified 3S motifs having any of SEQ ID NO: 156 to 160.

In other preferred embodiments, the modified polypeptides according to the invention are the following proteins having one or more modified 3S motif(s): UDP-glucose 4-epimerase, L-threonine 3-dehydrogenase, TonB-dependent receptor, DNA polymerase III subunit gamma/tau, Multidrug transporter AcrB, Peptide ABC transporter permease, Peptide ABC transporter substrate binding protein, MC73_05565, Chitinase, LVIVD repeat protein, AMK72_12855, HMPREF0183_2345, FAD-linked oxidoreductase, RNA-binding protein 48, Peptide ABC transporter, Type IV secretion protein Rhs, Putative transmembrane rhomboid family protein, Receptor kinase-like protein Xa21, Rim ABC transporter, Importin beta SMX1, Transporter, Sugar ABC transporter permease, ABC transporter substrate binding protein, Tetraspanin, Nucleotidyltransferase, phosphocholine-specific Phospholipase C, Response regulator of zinc sigma-54-dependent two-component system, Peptide ABC transporter permease, Peptide ABC transporter, Type IV secretion protein RhS, Importin beta SMX1, Nucleotidyltransferase.

Polypeptide modification can be performed by techniques known per se in the art, such as mutagenesis, chemical synthesis, enzymatic synthesis, recombinant technology, and the like, or combinations thereof.

### Specific embodiments of the invention

In a further particular embodiment, the polypeptide of the invention is a component of a pathogen. In a more specific embodiment, the polypeptide of the invention is a viral or bacterial or fungal or parasite protein or polypeptide.

In another specific embodiment, the polypeptide of the invention is a HIV gp41 polypeptide having one or more modified 3S motifs, or a fragment or mimotope thereof. In a particular embodiment, the polypeptide comprises or consists of SEQ ID NO: 3 with at least one modified 3S motif, or a fragment or mimotope thereof. Such polypeptide is particularly associated with HIV infection.

An example of the modified gp41 polypeptide according to the invention is shown below:

AAIGALFLGFLGAAGSTMGAASVTLTVQARLLLSGIVQQQNNLLRAIESQQHMLRLTVWGIKQLQARVLAVERYLK
DQQLLGFWGCSGKLICTTTVPWNAAWSNKSLDDIWNNMTWMQWEREIDNYTSLIYSLLEKSQTQQEKNEQELL
ELDKWASLWNWFDITNWLWYIKIFIMIVGGLVGLRIVFAVLSIVNRVRQGYSPLSLQTRPPVPRGPDRPEGIEEEGG
ERDRDTSGRLVHGFLAIIWVDLRSLFLLSYHHLRDLLLIAARIVELLGRRGWEVLKYWWNLLQYWSQELKSSAVSLLN
AAAIAVAEGTDRVIEVLQRAGRAILHIPTRIRQGLERALL (SEQ ID NO: 163)

In another specific embodiment, the polypeptide of the invention is a HIV gp120 polypeptide having one or more modified 3S motifs, or a fragment or mimotope thereof. In a particular embodiment, the polypeptide comprises or consists of any one of SEQ ID NO: 84 to 93 with at least one modified 3S motif, or a fragment or mimotope thereof. Such polypeptide is particularly associated with HIV infection.

An example of the modified gp120 polypeptide according to the invention is shown below:

LWVTVYYGVPVWKEATTTLFCASDAKAYDTEVHNVWATQACVPTDPNPQEVELVNVTENFNAWKNNMVEQM
HEDIISLWDQSLKPCVKLTPLCVTLNCTDLRNTTNTNNSTANNNSNSEGTIKGGEMKNCSFNITTSIRDKMQKEYAL
LYKLDIVSIDNDSTSYRLISCNTSVITQACPKISFEPIPIHYCAPAGFAILKCNDKKFSGKGSCKNVSTVQCTHGIRPVVST
QLLLNGSLAEEEVVIRSENFTDNAKTIIVHLNESVQINCTRPNYNKRKRIHIGPGRAFYTTKNIIGTIRQAHCNISRAKW
NDTLRQIVSKLKEQFKNKTIVFNQSSGGDPEIVMHSFNCGGEFFYCNTSPLFNSTWNGNNTWNNTTGSNNNITLQ

CKIKQIINMWQEVGKAMYAPPIEGQIRCSSNITGLLLTRDGGKDTDTNDTEIFRPGGGDMRDNWRSELYKYKVVTI
EPLGVAPTKAKRRVVQREKR (SEQ ID NO: 164)

In another specific embodiment, the polypeptide of the invention is a HIV gp160 polypeptide having one or more modified 3S motifs, or a fragment or mimotope thereof. In a particular embodiment, the polypeptide comprises or consists of any one of SEQ ID NO: 100 to 105 with at least one modified 3S motif, or a fragment or mimotope thereof. Such polypeptide is particularly associated with HIV infection.

An example of the modified gp160 polypeptide according to the invention is shown below:

MRVKETQMNWPNLWKWGTLILGLVIICSASDNLWVTVYYGVPVWRDADTTLFCASDAKAHETEVHNVWATHA
CVPTDPNPQEIDLENVTENFNMWANNMVEQMQEDVISLWDQSLKPCVKLTPLCVTLHCTNANLTKANLTNVNN
RTNVSNIIGNITDEVRNCSFNMTTELRDKKQKVHALFYKLDIVPIEDNNDNSKYRLINCNTSVIKQACPKISFDPIPIHY
CTPAGYAILKCNDKNFNGTGPCKNVSSVQCTHGIKPVVSTQLLLNGSLAEEEIIIRSEDLTNNAKTIIVHLNKSVVINCT
RPSNNTRTSITIGPGQVFYRTGDIIGDIRKAYCEINGTEWNKALKQVTEKLKEHFNNKPIIFQPPSGGDLEITMHHFNC
RGEFFYCNTTRLFNNTCIANGTIEGCNGNITLPCKIKQIINMWQGAGQAMYAPPISGTINCVSNITGILLTRDGGATN
NTNNETFRPGGGNIKDNWRNELYKYKVVQIEPLGVAPTRAKRRVVEREKRAVGIGAMIFGFLGAAGSTMGAASITL
TVQARQLLSGIVQQQSNLLRAIEAQQHLLQLTVWGIKQLQARVLAVERYLKDQKFLGLWGCSGKIICTTAVPWNST
WSNKSLEEIWNNMTWIEWEREISNYTNQIYEILTKSQDQQDRNEKDLLELDKWASLWTWFDITNWLWYIKIFIMI
VGGLIGLRIIFAVLSIVNRVRQGYSPLSFQTPCHHQREPDRPERIEEEGGEQGRDRSVRLVSGFLALAWDDLRSLCLFS
YHRLRDFILIAARTVELLGRSSLKGLRRGWEGLKYLGNLLLYWGQELKISAISLLDATAIAVAGWTDRVIEVAQGAWK
AILHIPRRIRQGLERALQ (SEQ ID NO: 165)

In another specific embodiment, the polypeptide of the invention is a HIV gp140 polypeptide having one or more modified 3S motifs, or a fragment or mimotope thereof. In a particular embodiment, the polypeptide comprises or consists of any one of SEQ ID NO: 94 to 99 with at least one modified 3S motif, or a fragment or mimotope thereof. Such polypeptide is particularly associated with HIV infection.

An example of the modified gp140 polypeptide according to the invention is shown below:

LWVTVYYGVPVWKEATTTLFCASDAKAYDTEVHNVWATHACVPTDPNPQEVVLVNVTENFNMWKNDMVEQM
HEDIISLWDQSLKPCVKLTPLCVSLKCTDLKNDTNTNSSSGRMIMEKGEIKNCSFNISTSIRGKVQKEYAFFYKLDIIPID
NDTTSYKLTSCNTSVITQACPKVSFEPIPIHYCAPAGFAILKCNNKTFNGTGPCTNVSTVQCTHGIRPVVSTQLLLNGS
LAEEEVVIRSVNFTDNAKTIIVQLNTSVEINCTRPNNNTRKRIRIQRGPGRAFVTIGKIGNMRQAHCNISRAKWNNTL
KQIASKLREQFGNNKTIIFKQSSGGDPEIVTHSFNCGGEFFYCNSTQLFNSTWFNSTWSTEGSNNTEGSDTITLPCRI
KQIINMWQKVGKAMYAPPISGQIRCSSNITGLLLTRDGGNSNNESEIFRPGGGDMRDNWRSELYKYKVVKIEPLGV
APTKAKRRVVQREKRAVGIGALFLGFLGAAGSTMGAASMTLTVQARQLLSGIVQQQNNLLRAIEAQQHLLQLTVW
GIKQLQARILAVERYLKDQQLLGIWGCSGKLICTTAVPWNAAWANKSLEQIWNHTTWMEWDREINNYTSLIHSLIE
ESQNQQEKNEQELLELDKWASLWNWFNITNWLWYI (SEQ ID NO: 166)

In another specific embodiment, the polypeptide of the invention is a Staphylococcus aureus protein A having one or more modified 3S motifs, or a fragment or mimotope thereof. In a particular embodiment, the polypeptide comprises or consists of SEQ ID NO: 44 with at least one modified 3S motif, or a mimotope or fragment thereof. Such polypeptide is particularly associated with S. aureus infection.

An example of the modified S. aureus polypeptide according to the invention is shown below:

MKKKNIYSIRKLGVGIASVTLGTLLISGGVTPAANAAQHDEAQQNAFYQVLNMPNLNADQRNGFIQSLKD
DPSQSANVLGEAQKLNDSQAPKADAQQNNFNKDQQSAFYEILNMPNLNEAQRNGFIQSLKDDPSQSTNVL
GEAKKLNESQAPKADNNFNKEQQNAFYEILNMPNLNEEQRNGFIQSLKDDPSQSANLLSEAKKLNESQAP
KADNKFNKEAANAFYEILHLPNLNEEQRNGFIQSLKDDPSQSANLLAEAKKLNDAQAPKADNKFNKEQQN
AFYEILHLPNLTEEQRNGFIQSLKDDPSVSKEILAEAKKLNDAQAPKEEDNNKPGKEDNNKPGKEDNNKP
GKEDNNKPGKEDNNKPGKEDGNKPGKEDNKKPGKEDGNKPGKEDNKKPGKEDGNKPGKEDGNKPGKEDGN
GVHVVKPGDTVNDIAKANGTTADKIAADNKLADKNMIKPGQELVVDKKQPANHADANKAQALPETGEENP
FIGTTVFGGLSLALGAALLAGRRREL (SEQ ID NO: 167)

In another specific embodiment, the polypeptide of the invention is a P. gingivalis polypeptide having one or more modified 3S motifs, or a fragment or mimotope thereof. In a particular embodiment, the polypeptide consists of SEQ ID NO: 2 with at least one modified 3S motif, or a fragment or mimotope thereof. Such polypeptide may for example comprise a 3S motif of SEQ ID NO: 7 or 8, wherein at least one amino acid has been modified, preferably substituted, more preferably substituted by alanine. Such polypeptide is particularly associated with P. gingivalis infection.

In a specific embodiment, the polypeptide of the invention is a HCV core polypeptide having one or more modified 3S motifs, or a fragment or mimotope thereof. In a particular embodiment, the polypeptide comprises or consists of SEQ ID NO: 43 with at least one modified 3S motif, or a mimotope or fragment thereof. Such polypeptide is particularly associated with HCV infection.

In another specific embodiment, the polypeptide of the invention is a Sars-CoV-2 protein A having one or more modified 3S motifs, or a fragment or mimotope thereof. In a particular embodiment, the polypeptide comprises a 3S motif of any of SEQ ID NO: 141 to 145, wherein at least one amino acid has been modified, preferably substituted, more preferably substituted by alanine. Such polypeptide is particularly associated with Sars-CoV-2 infection.

In another specific embodiment, the polypeptide of the invention is a P. mirabilis polypeptide having one or more modified 3S motifs, or a fragment or mimotope thereof. In a particular embodiment, the polypeptide comprises a 3S motif of SEQ ID NO: 14, wherein at least one amino acid has been modified, preferably substituted, more preferably substituted by alanine. Such polypeptide is particularly associated with P. mirabilis infection.

In another specific embodiment, the polypeptide of the invention is a L. weilii str. polypeptide having one or more modified 3S motifs, or a fragment or mimotope thereof. In a particular embodiment, the polypeptide comprises a 3S motif of SEQ ID NO: 18, wherein at least one amino acid has been modified, preferably substituted, more preferably substituted by alanine. Such polypeptide is particularly associated with L. weilii str. infection.

In another specific embodiment, the polypeptide of the invention is a T. glycolicus polypeptide having one or more modified 3S motifs, or a fragment or mimotope thereof. In a particular embodiment, the polypeptide comprises a 3S motif of SEQ ID NO: 28, wherein at least one amino acid has been modified, preferably substituted, more preferably substituted by alanine. Such polypeptide is particularly associated with T. glycolicus infection.

In another specific embodiment, the polypeptide of the invention is a B. fragilis polypeptide having one or more modified 3S motifs, or a fragment or mimotope thereof. In a particular embodiment, the polypeptide comprises a 3S motif of SEQ ID NO: 29 or 30, wherein at least one amino acid has been modified, preferably substituted, more preferably substituted by alanine. Such polypeptide is particularly associated with B. fragilis infection.

In another specific embodiment, the polypeptide of the invention is a C. glabrata polypeptide having one or more modified 3S motifs, or a fragment or mimotope thereof. In a particular embodiment, the polypeptide comprises a 3S motif of SEQ ID NO: 33, wherein at least one amino acid has been modified, preferably substituted, more preferably substituted by alanine. Such polypeptide is particularly associated with C. glabrata infection.

In another specific embodiment, the polypeptide of the invention is a A. actinomycetemcomitans infection polypeptide having one or more modified 3S motifs, or a fragment or mimotope thereof. In a particular embodiment, the polypeptide comprises a 3S motif of SEQ ID NO: 38, wherein at least one amino acid has been modified, preferably substituted, more preferably substituted by alanine. Such polypeptide is particularly associated with A. actinomycetemcomitans infection.

In another specific embodiment, the polypeptide of the invention is a P. somerae infection polypeptide having one or more modified 3S motifs, or a fragment or mimotope thereof. In a particular embodiment, the polypeptide comprises a 3S motif of SEQ ID NO: 41, wherein at least one amino acid has been modified, preferably substituted, more preferably substituted by alanine. Such polypeptide is particularly associated with P. somerae infection.

In another specific embodiment, the polypeptide of the invention is a A. aphrophilus infection polypeptide having one or more modified 3S motifs, or a fragment or mimotope thereof. In a particular embodiment, the polypeptide comprises a 3S motif of SEQ ID NO: 42, wherein at least one amino acid has been modified, preferably substituted, more preferably substituted by alanine. Such polypeptide is particularly associated with A. aphrophilus infection.

Further illustrative examples of polypeptides of the invention are molecules or agents in the plasma of cancer patients, or variants or derivatives thereof, which can exert a cofactor effect on PLA2G1B.

Each of the above polypeptide, or a fragment or a variant thereof, may represent an antigen of interest in the context of a prophylactic or therapeutic vaccine according to the invention.

### Nucleic acids, vectors and host cells

The invention also relates to nucleic acids encoding polypeptides of the invention, any vector comprising such a nucleic acid, recombinant host cells containing such a nucleic acid or vector, and the uses thereof.

The nucleic acid according to the invention may be an isolated or purified nucleic acid. The nucleic acid can be DNA (cDNA or gDNA), RNA, or a mixture thereof. The nucleic acid can be single-stranded or double-stranded or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the polypeptide according to the invention and codon usage may be adapted according to the host cell in which the nucleic acid shall be transcribed. These steps may be carried out according to methods well known to one of skill in the art and some of which are described in the reference manual Sambrook et al. (Sambrook J, Russell D (2001) Molecular cloning: a laboratory manual, Third Edition Cold Spring Harbor).

The present invention further relates to a vector comprising a nucleic acid of the invention. Optionally, the vector may comprise several nucleic acids of the invention. In particular, the vector may comprise a nucleic acid of the invention operably linked to a regulatory region, i.e. a region comprising one or more control sequences. Optionally, the vector may comprise several nucleic acids of the invention operably linked to several regulatory regions. The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal peptide sequence and transcription terminator. The term "operably linked" means a configuration in which a control sequence is placed at an appropriate position relative to a coding sequence, in such a way that the control sequence directs expression of the coding region. The present invention further relates to the use of a nucleic acid or vector according to the invention to transform, transfect or transduce a host cell.

The vector according to the invention may be any vector such as for example, a viral vector (e.g., adenovirus, adeno-associated virus, lentivirus, retrovirus, canarypox, etc.), a plasmid, a phage, an artificial chromosome, etc.

The present invention also provides a host cell comprising one or several nucleic acids of the invention and/or one or several vectors of the invention. The term "host cell" also encompasses any progeny of a parent host cell that is not identical to the parent host cell due to mutations that occur during replication.

Suitable host cells for cloning or expression of antibody-encoding vectors include prokaryotic or eukaryotic cells as known in the art.

The present invention also concerns a method for producing a modified polypeptide of the invention, comprising culturing a host cell comprising a nucleic acid of the invention or a vector of the invention, as provided above, under conditions suitable for expression of the polypeptide, and optionally recovering the polypeptide from the host cell or from the host cell culture medium. Optionally, the recovered antibody may be further purified or isolated. Suitable media, culture conditions and production method are well-known by the skilled person and can be easily chosen according to the host cell and the antibody to be produced.

### Vaccine compositions and methods

The present invention also relates to vaccine compositions comprising a modified polypeptide, nucleic acid, vector, or cell, according to the invention. The vaccine compositions may be for prophylactic or therapeutic treatment.

In a preferred embodiment, the vaccine according to the invention comprises a modified polypeptide comprising one or more modified 3S motif(s), as described herein.

In a particular embodiment, the vaccine according to the invention comprises a modified polypeptide comprising one or more modified 3S motif(s) having any of SEQ ID NO: 45 to 71 or 146 to 160, as described in the experimental part.

The vaccine may be a subunit, vectorized, DNA vaccine or plasmid-mediated vaccine, etc.

Vaccine may contain several antigens, for combined, separate or sequential administration. For instance, the vaccine may contain a vectorized vaccine and a subunit vaccine; or a DNA plasmid vaccine and a vector vaccine; or a DNA plasmid vaccine and a viral vector vaccine and a protein, etc.

For subunit, the antigen may be monomeric or multimeric (e.g., dimeric, trimeric, tetrameric, etc.), free or conjugated, optionally linked or mixed with a carrier, etc.

The antigen of the invention comprises or consists of a modified polypeptide according to the invention.

The antigen may be in various forms such as in free form, polymerized, chemically or physically modified, and/or coupled (i.e., linked) to a carrier molecule. Coupling to a carrier may increase the immunogenicity and (further) suppress the biological activity of the polypeptide according to the invention. In this regard, the carrier molecule may be any carrier molecule or protein conventionally used in immunology such as for instance KLH (Keyhole limpet hemocyanin), ovalbumin, bovine serum albumin (BSA), a viral or bacterial anatoxin such as toxoid tetanos, toxoid diphteric B cholera toxin, mutants thereof such as diphtheria toxin CRM 197, an outer membrane vesicle protein, a polylysine molecule, or a virus like particle (VLP). Coupling of the antigen to a carrier may be performed by covalent chemistry using linking chemical groups or reactions, such as for instance glutaraldehyde, biotin, etc.

In this regard, in a particular embodiment, the vaccine of the invention comprises a modified polypeptide of the invention, optionally coupled to a carrier protein. In a preferred embodiment, the antigen comprises a modified polypeptide comprising one or more modified 3S motif(s) having any of SEQ ID NO: 45 to 71 or 146 to 160, coupled to a carrier protein.

The immunogenicity of the antigen according to the invention may be tested by various methods, such as by immunization of a non-human animal grafted with human immune cells, followed by verification of the presence of antibodies, or by sandwich ELISA using human or humanized antibodies.

In another embodiment, the invention relates to a modified immunogenic polypeptide, wherein said polypeptide comprises one or more modified 3S motif(s) as described herein, and wherein said modified polypeptide has reduced PLA2G1B cofactor effect on CD4 T cell as compared to a corresponding polypeptide without said modified 3S motif(s).

In a further embodiment, the invention relates to a vaccine comprising a modified polypeptide according to the invention, in combination with a suitable excipient and, optionally, a suitable adjuvant.

A further object of the invention relates to a method for preparing a vaccine comprising an antigen, the method comprising (i) modifying a 3S motif present in the antigen, and (ii) formulating the antigen as a vaccine.

A further object of the invention relates to a method for preparing an antigen, comprising (i) selecting an antigen of interest, and (ii) modifying a 3S motif present in the antigen so as to reduce a PLA2G1B cofactor effect of the antigen.

A further object of the invention relates to a method of optimizing an antigen of the invention, comprising modifying at least one 3S motif and verifying PLA2G1B cofactor effect, and optionally verifying the immunogenicity of the antigen.

In this regard, the inventors have developed an assay to directly follow PLA2G1B enzymatic activity on CD4 T-cell membrane. The inventors demonstrated that gp41 directly binds to PLA2G1B and increases PLA2G1B enzymatic activity on CD4 membrane. Furthermore, using gClqR KO cells, the inventors showed that the conserved 3S sequence of gp41, known to bind to gC1qR, increased PLA2G1B activity in a gC1qR-dependent manner. By screening additional microbial proteins for 3S-like motif and studying other proteins known to bind to gC1qR, the inventors extended their observation in the case of HIV-infection to other pathologies where microbial proteins with 3S-like motif or binding to gClqR also increased PLA2G1B enzymatic activity. The inventors identified a mechanism of regulation of CD4 T-cell function through the regulation of PLA2G1B activity via gClqR and cofactors which are disease specific and gClqR binding proteins that can contain 3S-like motif. This mechanism is involved in HIV-1 immunodeficiency and could play a role in other immune diseases and cancers.

Administration of an antigen or vaccine of the invention may be by any suitable route, such as by injection, preferably intramuscular, subcutaneous, transdermal, intravenous or intraarterial, or by nasal, oral, mucosal or rectal administration.

The invention may be used in any mammal, particularly in human subjects. The invention is in particular suitable to treat diseases wherein a stimulation or restoration of CD4 T cells is beneficial, such as diseases caused by infectious pathogens (e.g., virus or bacteria) or by undesirable cell proliferation (e.g., cancers).

The modified polypeptide or vaccine of the invention may be used for treating any disease associated with a CD4 T cell immunodeficiency. More specifically, this invention relates to a method for preventive or curative treating a disease associated with a CD4 T cell immunodeficiency in a subject in need thereof, comprising administering to the subject a vaccine composition comprising a modified polypeptide, wherein said polypeptide comprises a modified 3S motif, and wherein said modified polypeptide has reduced PLA2G1B cofactor effect on CD4 T cell as compared to a corresponding polypeptide without said modified 3S motif.

The modified polypeptide or vaccine of the invention is suitable for treating any disease or disorder by inducing or stimulating an antigen-specific immune response.

The modified polypeptide or vaccine of the invention is particularly suitable for treating any infectious disease such as a viral or bacterial disease, etc.

In a particular embodiment, the modified polypeptide of the invention is for use in the treatment of AIDS.

In another particular embodiment, the modified polypeptide of the invention is for use for vaccinating against HIV.

In another particular embodiment, the modified polypeptide of the invention is for use for vaccinating against HCV.

In another particular embodiment, the modified polypeptide of the invention is for use for vaccinating against S. aureus.

The modified polypeptide of the invention is also suitable for treating cancer.

A particular object of the invention relates to the modified polypeptide as described herein, for use in a method of vaccinating a mammal against a disease associated with a CD4 T cells immunodeficiency such as in particular an HIV infection.

The vaccines and methods of the invention are useful to immunize subjects, thereby causing a reduced PLA2G1B cofactor effect on CD4 T cells. Upon repetition, such vaccines can be used to cause a permanent inhibition of PLA2G1B cofactor effect on CD4 T cells. As a result of such a vaccination, the subject produces antibodies (or cells) which induce an increased immune response after administration of such antigens comprising modified 3S-like sequences.

### Examples

### MATERIALS AND METHODS

### Recombinant proteins and peptides

Human PLA2G1B was produced in E. coli (gift Gerard Lambeau, purity >98%) or in CHO-S (purity >98%). gp120, gp41 and gp140 proteins were produced in S2 cells or HEK 293 T cells. All synthetic peptides were and will be ordered from Covalab (purity >98%): MN-3S peptide NH2-PWNASWSNKSLDDIW-COOH and control peptide (CTL) NH2-PWNATWTQRTLDDIW-COOH, Scrambled MN-3S NH2-WNWDSKILSDPAWNS-COOH peptides 3S-like peptide from P. gingivalis HP Pg NH2-SGEGGWSNGSLVDIM-COOH. ZM96C-3S homologous peptide NH2-PWNISWSNKSKTDIW-COOH scrambled ZM96C-3S NH2-WNWKSKIDSTPIWNS-COOH and 3S-like peptides NH2-ENFNMWKNDMVDQMH-COOH, NH2-SWNNLWNWFDITKWL-COOH, NH2-NNLWNWFDITKWLWY-COOH, NH2-GWEALKYLGSLVQYW-COOH in gp160 subtype C (ZM96C, envelope glycoprotein HIV strain HIV-1 96ZM651, from UniprotKB #Q994Q6) from ALVAC HIV (vCP2438).

BSA was order from sigma (fatty acid free A6003-10G). HCV core protein was obtained from Prospec (HCV-011, purity >95%) in PBS buffer with 0.002%SDS and the specificity of effect due to HCV core protein was evaluated by comparison with similar dilution of PBS SDS 0.002%). Staphylococcus aureus protein A was obtained from Sigma (P6031).

### Purification of Human CD4 T-lymphocytes

Venous blood was obtained from healthy volunteers through the EFS (Etablissement Français du Sang, Centre Necker-Cabanel, Paris). CD4 T-cells were purified from whole blood using RosetteSep Human CD4+ T cell Enrichment Cocktail (Stem Cell, 15062). This cocktail contains mouse and rat monoclonal antibodies purified from mouse ascites fluid or hybridoma culture supernatant, by affinity chromatography using protein A or Protein G sepharose. These antibodies are bound in bispecific tetrameric antibody complexes which are directed against cell surface antigens on human hematopoietic cells (CD8, CD16, CD19, CD36, CD56 CD66b, TCRγ/δ and glycophorin A on red blood cells. The rosetteSep antibody cocktail crosslinks unwanted cells in human whole blood to multiple red blood cells, forming immunorosettes. This increases the density of unwanted cells, such that they pellet along with the free red blood cells when centrifuged over a buoyant density medium such as lymphocytes separation medium (Eurobio, CMSMSL01-01).

Whole blood was incubated with RosetteSep Human CD4+ T cell Enrichment Cocktail at 50µl/ml for 20 minutes at room temperature under gentle shaking (100 rpm), diluted with an equal volume of PBS+2% foetal bovine serum (FBS) and mixed gently. The diluted samples were centrifuged 20 minutes at 1200 X g on top of lymphocytes separation medium. The enriched cells were then collected from the density medium at plasma interface and washed twice with PBS+2% FBS. Cells were subsequently resuspended in RPMI 1640 medium (Lonza) supplemented with 5% FBS, 50 mM HEPES pH 7.4, glutamine, penicillin, streptomycin and fungizone (complete medium), counted with a Moxi Z mini automated cell counter (ORFLO, MXZ000). Cell suspension was adjusted at 7×10⁶ cells/ml and equilibrated at least 2 h at 37°C in a 5% CO2 humidified atmosphere.

The enriched CD4-T cell population was controlled by flow cytometry on a cytoflex (Beckman coulter). The quiescence of recovered CD4 T-cells was controlled by the low level of IL-2Rα (CD25). CD4 T cells were labeled with anti-Human CD3 eFluor780 (eBioscience, clone UCHT1, 47-0038-42), anti-Human CD25-PE (Biolegend, clone BC96, 302605) and anti-human CD4-PerCP (BD, clone SK3, 345770). The enriched CD4-T cell population contains >95% CD3+CD4+ and less than 8% of CD25+.

### PLA2G1B enzymatic assay on [3H] arachidonic acid labelled CD4 T cells

Purified CD4 T-cells were incubated for 16h at 2×10⁶ cells/ml with 1µCi/ml of arachidonic acid [5,6,8,9,11,14,15-³H(N)] (Perkin Elmer, NET298Z250UC) in RPMI 1640 medium (Lonza) supplemented with 10% FBS, 50 mM HEPES pH 7.4, glutamine, penicillin, streptomycin and fungizone at 2 ml/well in 6-well plates at 37°C in a 5% CO2 humidified atmosphere. Cells were washed twice with RPMI with 10% FBS by centrifugation at 580xg for 10 minutes at room temperature and then frozen in 90% FBS 10% DMSO at 10⁷ cells/ml/vial at -80°C. Percent of [3H] arachidonic acid in CD4 T cells is the (1 minus ratio of [3H] arachidonic acid in the supernatant of CD4 T cells without cells (cpm/ml) on total [3H] arachidonic acid in supernatant and cells (cpm/ml).

To test PLA2G1B activity on [3H] arachidonic acid labelled CD4 T lymphocytes, cells were unfrozen in 10% FBS RPMI preheated at 37°C by centrifugation at 580xg for 10 minutes at room temperature, washed twice in 2.5% FBS RPMI, and equilibrated at 2×10⁵ CD4 T cells/400µl/well in 24-well polystyrene plates for 1h30 h at 37°C in a 5% CO2 humidified atmosphere. Then 100µl of recombinant proteins (HIV-1 gp140, gp120, gp41, HCV core protein, HCV-011, Prospec, Staphylococcus aureus protein A, P6031, Sigma) or vehicle dilution with or without PLA2G1B in 2.5% FBS RPMI was added to each well for 2h. Cells and supernatant were collected in eppendorf tubes and centrifuged at 580 x g for 10 minutes at room temperature. The [3H] arachidonic acid released in cell supernatant was quantified in 300µ1 with 16 ml of Ultima gold (Perkin Elmer, 6013329) in low diffusion vials (Perkin Elmer, 6000477) on a counter (tri-Carb 2800 TR liquid scintillation analyzer, Perkin Elmer).

### PLA2G1B enzymatic assay on [3H] arachidonic acid labelled Jurkat E6.1 T cells

Jurkat E6.1 T cells (ECACC 88042803) or gClqR KO Jurkat E6.1 T cells were incubated for 17h at 5×10⁵ cells/ml with 1µCi/ml of arachidonic acid [5,6,8,9,11,14,15-³H(N)] (Perkin Elmer, NET298Z250UC) in RPMI 1640 medium (Lonza) supplemented with 10% FBS, 50 mM HEPES pH 7.4, glutamine, penicillin, streptomycin and fungizone at 2 ml/well in 6-well plates at 37°C in a 5% CO2 humidified atmosphere. Cells were washed twice with RPMI with 10% FBS by centrifugation at 300xg for 10 minutes at room temperature and then frozen in 90% FBS 10% DMSO at 10⁷ cells/ml/vial at -80°C. Percent of [3H] arachidonic acid in CD4 T cells is the (1 minus ratio of [3H] arachidonic acid in the supernatant of CD4 T cells without cells (cpm/ml) on total [3H] arachidonic acid in supernatant and cells (cpm/ml).

To test PLA2G1B activity on [3H] arachidonic acid labelled Jurkat E6.1 T lymphocytes, cells were unfrozen in 10% FBS RPMI preheated at 37°C by centrifugation at 300xg for 10 minutes at room temperature, washed twice in 2.5% FBS RPMI, and equilibrated at 5×10⁴ or 10⁵ Jurkat E6.1 T cells/400µl/well in 24-well polystyrene plates for 1h30 at 37°C in a 5% CO2 humidified atmosphere. Cells were pretreated with peptides for 2h, 4h or 21h, as indicated on figures, with 50µl per well of peptide solutions at 110µM or 55µM in 2.5% FBS RPMI. Then 50µl per well of PLA2G1B at 630nM or 2µM 2.5% FBS RPMI or medium alone were added for 2h. Cells and supernatant were collected in eppendorf tubes and centrifuged at 580xg for 10 minutes at room temperature. The [3H] arachidonic acid released in cell supernatant was quantified in 300µ1 with 16 ml of Ultima gold (Perkin Elmer, 6013329) in low diffusion vials (Perkin Elmer, 6000477) on a counter (tri-Carb 2800 TR liquid scintillation analyzer, Perkin Elmer).

Results are expressed as PLA2G1B activity (release of [3H] arachidonic acid in the supernatant of cells treated with peptide or HCV core together with PLA2G1B minus spontaneous release of [3H] arachidonic acid by cells with peptide or buffer only without PLA2G1B in cpm/ml) or ΔPLA2G1B activity with peptides minus activity with Scrambled MN-3S peptide (release of [3H] arachidonic acid in the supernatant of cells treated with peptide minus release of [3H] arachidonic acid by cells treated with Scrambled MN-3S in cpm/ml).

To test PLA2G1B activity on [3H] arachidonic acid labelled Jurkat E6.1 T lymphocytes, cells were unfrozen in 10% FBS RPMI preheated at 37°C by centrifugation at 300xg for 10 minutes at room temperature, washed twice in 2.5% FBS RPMI, and equilibrated at 5×10⁴ or 10⁵ Jurkat E6.1 T cells/400µl/well in 24-well polystyrene plates for 1h30 at 37°C in a 5% CO2 humidified atmosphere. For peptide treatments, cells were pretreated for 21h with 50µl per well of peptide solutions at 110µM or 55µM in 2.5% FBS RPMI. Then 50µl per well of PLA2G1B at 630nM or 2µM 2.5% FBS RPMI or medium alone were added for 2h. Cells and supernatant were collected in eppendorf tubes and centrifuged at 580xg for 10 minutes at room temperature. The [3H] arachidonic acid released in cell supernatant was quantified in 300µ1 with 16 ml of Ultima gold (Perkin Elmer, 6013329) in low diffusion vials (Perkin Elmer, 6000477) on a counter (tri-Carb 2800 TR liquid scintillation analyzer, Perkin Elmer).

Results are expressed as PLA2G1B activity (release of [3H] arachidonic acid in the supernatant of cells treated with peptide together with PLA2G1B minus spontaneous release of [3H] arachidonic acid by cells in cpm/ml) or ΔPLA2G1B activity with peptides minus activity with Scrambled peptide (release of [3H] arachidonic acid in the supernatant of cells treated with peptide minus release of [3H] arachidonic acid by cells treated with Scrambled in cpm/ml).

### Detection of antibody against gp41 and gp120 recombinant proteins by ELISA.

Recombinant WT or mutant gp41, gp120 were produced in S2 cells. Microtiter plates are coated in triplicate with 10 µg/well of either recombinant gp120 or recombinant gp41 in carbonate buffer pH 9.6. After overnight incubation at 4°C, plates are washed 3 times with 250 µl/well of PBS with 0.1% Tween 20 (PBST) and nonspecific binding sites are blocked with 100 µl/well of 3% non-fat milk prepared in PBST (1h, RT). Blocking solution is removed and 100 µl/well of mouse or monkey plasma diluted in PBST with 1% non-fat milk with plasma dilution starting at 1:25, with five serial 3-fold dilutions for IgG detection and six serial 4-fold dilutions for IgM detection, and of each serial in duplicate will be added for 2h at RT. After washing, bound antibody are then detected by interaction with anti-mouse IgG or anti-mouse IgM-HRP or anti-macaque IgG-HRP or anti-macaque IgM-HRP diluted in dilution in PBST with 1% non-fat milk for 1h RT is revealed by the addition of 100 µl/well TMB substrate (UP664781, Interchim).

### ELISA study of PLA2G1B and cofactors interaction

Study of gp41, gp120 or gp140 binding to coated PLA2G1B in ELISA. Microplates were coated with 100µl of PLA2G1B recombinant proteins at 10 µg/mL in blocking buffer (Pierce). Then binding of serial dilutions (0-1µg/well) of strep-tagged gp41, gp120, gp140, or unrelated proteins (CTL1: EFF-1 or CTL2: IF38) in triplicate, to PLA2G1B was revealed by sequential addition of mouse anti-strep-tag mAb (MA5-17283, Invitrogen), goat anti-mouse HRP-conjugated Ab (31430, Pierce) and HRP reaction with TMB.

Study of PLA2G1B binding to coated gp41, gp120 or gp140 in ELISA. Microplates were coated with 100µl of gp41, gp120 or gp140 recombinant proteins at 10 µg/mL in blocking buffer (Pierce). Then binding of serial dilutions (0-1µg/well) of PLA2G1B in triplicate, to gp41, gp120 or gp140 was revealed by sequential addition of anti-PLA2G1B mAb (1C11), HRP-anti-mouse Ab and HRP reaction with TMB.

### Coimmunoprecipitation assay of PLA2G1B with cofactors

Recombinant gp41, gp120 or gp140 with a strep-tag at 10 µg/mL in PBS was incubated or not with recombinant PLA2G1B (50 µg/mL) in 1.5-ml Eppendorf tubes overnight (Test tube-rotor, 34528, Snijders, Netherland) at 4°C. Then gp41 was immunoprecipitated by adding 50 µL/tube of prewashed streptactin-beads (Mag strep "type 3" XP beads, IBA, 2-4090-002) followed by an incubation for 30 min on ice with vortexing from time to time (4X). Beads were washed 3 times in washing buffer and immunoprecipitated protein complex were eluted in 50µL of BTX buffer 1X (IBA, 2-1042-025). 5 µL of flow through and 30 µL of eluted sample were loaded on 4%-20% Tris-Bis SDS-PAGE (BIO-RAD) gels under reducing conditions. Antigens were transferred to PVDF membranes (BIO-RAD) using a Trans-Blot Turbo (BIO-RAD. After blocking nonspecific binding sites with 5% milk/0.05% tween 20 in PBS, PLA2G1B was revealed with 10 µg/ml for 1C11 mouse anti-PLA2G1B mAb, gp41 or gp140 were revealed with 5 µg/ml of polyclonal anti-gp41 (PA1-7219, Thermofisher) and gp120 was revealed with anti-gp120 Ab. Goat anti-mouse (31430, Pierce) and donkey anti-goat (705-035-003, Jackson Immunoresearch) HRP-conjugated antibodies were used at a 1:10,000 dilution. Detection occurred directly on the membrane using SuperSignal West Pico Plus Substrate (34580, Thermo Fisher Scientific).

### Generation of gC1qR KO Jurkat E6.1 T cells

The global strategy for the development of Jurkat cells deprived of C1QBP is based on the design of a targeting vector permitting bi-allelic inactivation of C1QBP gene via homologous recombination. Human C1QBP homologous regions isogenic with the Jurkat E6.1T cell line (ECACC 88042803) has been used. The targeting vector has been synthetized by Genewiz and cloned into the pUC57-Amp vector. The third exon of human C1QBP gene was targeted by introducing a neomycin resistance gene (NeoR) selection cassette, this results in the interruption of the C1QBP open reading frame. The NeoR cassette was cloned using BamHI /Notl restriction sites. The targeting vector has been verified by DNA restriction digestion cut with selected restriction enzymes (APaL1, Drd1, Pvu1, Pvu2, BamH1/Notl, Not1/Ncol, NEB) and target region sequencing. The DNA primers corresponding to C1QBP sgRNA (1828-Crispr_1A: CACC-GAAGTGACCGTGATTCTAAAA and 1828-Crispr_1B: AAAC-TTTTAGAATCACGGTCACTTC) were hybridized and cloned (Quick Ligase - New England Biolabs, NEB) into the pX330 plasmid (Addgene, 42230; Feng Zhang, MIT) using Bbsl restriction site (NEB).

The Jurkat cells (5 × 10⁶) were resuspended in 100 µL of Opti-MEM and 7µg of CRISPR/Cas9 plasmid and 2.5µg of targeting vector were added. The cells were electroporated with a Nepa21 electroporator. After cell selection in G418 selective medium, the Jurkat cell clones were prescreened by PCR genotyping. Independent cell clones knocked-out for C1QBP gene were amplified and verified by PCR genotyping and target region sequencing. Our validation pipeline for the independent Jurkat cell clones deficient for C1QBP gene consisted of PCR genotyping. The genomic DNA of gene edited Jurkat cells was isolated by proteinase K treatment and phenol purification. Each cell clone with bi-allelic inactivation of C1QBP gene was confirmed by PCR genotyping and by target region sequencing. PCR amplification was performed with Platinum HiFi Taq (Life technologies) for 2 min at 50°C with primers 1828_RH5_F: TACTACAGCCCTTGTTCTT and 1828_RH3_R: AGCACTTCCTGAAATGTT. The primers are designed in the C1QBP human locus and out of homologous arms. The WT and mutant allele are distinguished in the same PCR reaction. The wild type and mutant allele give 1146-bp and 2362-bp amplification product, respectively. This PCR genotyping protocol allows the identification of the homozygous Jurkat cell clones knocked-out for both alleles of C1QBP gene. The gene disruption in Jurkat cell line was achieved using CRISPR/Cas9 technology. The homozygous Jurkat cell clones deficient for C1QBP gene were obtained and validated by PCR genotyping and target region sequencing.

### Immunoblot detection of gC1qR in Jurkat E6.1 T cells

Western-blot analysis of gClqR protein expression in WT and gClqR KO Jurkat E6.1 T cells lysates. Cells were lysed in mammalian protein extraction reagent (M-PER, 11884111, Thermo Scientific) buffer and protein amount were quantified with BCA Protein assay kit on cleared supernatant (23227, Pierce, Thermo Scientific). An equal amount of total proteins was loaded for WT and gClqR KO Jurkat E6.1 T cells (40 µg) and fractionated by SDS-PAGE on Mini-PROTEAN TGX Stain Free Gels 8-16% (4568104, BIORAD), further electrotransferred, and probed by immunoblotting using a specific antibody against gClqR (60.11 Santa Cruz at 1:50, 74.5.2 Abcam at 1:1000) or β-actin (AC-74, Sigma at 1:2000) in PBS-Tween 0,05% BSA 5% at room temperature for 2h and a goat anti-mouse-IgG-HRP (1:20000, 31430, Invitrogen) in PBS PBS-Tween 0,05% BSA 5% for 1h. Bound antibodies were detected using ECL immunoblotting detection system (NEL103001EA, PerkinElmer).

### Study of 3S-like microbial peptide binding to gC1qR in solid-phase microplate binding assay

The ability of gClqR protein to bind to 3S-like microbial peptides was assessed by solid-phase microplate binding assay. 100µl of either 1mg/ml BSA, 2µg/ml of gp41, gp120 or gp140 or 5µg/ml of 3S-like peptides diluted in carbonate buffer, pH 9.6 (15 mM Na2CO3and 35 mM NaHCO3) were coated (in duplicates) overnight at 4°C on Nunc Maxisorp flat-bottom microplate (44-2404-21, Thermofisher Scientific). The unbound proteins or peptides were removed; the wells washed 2x with TBST (20mM Tris-HCI pH 7.5, 150mM NaCl, and 0.05% Tween-20) and the unreacted sites blocked by incubation (1 h, 37°C) with 300µl of 1% BSA in TBS previously heat inactivated (30 min, 56º C) and microfiltered (through a molecular sieve with a molecular weight cut off of 3500-5000). After washing (2x with TBST), the microtiter plate bound 3S-like peptide was incubated (1 h, 37°C) with 100 µl/well of biotinylated gClqR at 1µg/ml. After washing (2x with TBST), binding of gClqR to peptides was revealed by sequential reaction with AP-Neutravidin (Pierce 31002, #HB 98661, 1 h, 37°C), three washes with TBST, followed by p-nitrophenyl phosphate (pNPP, Thermofisher 34045, 100mM Tris pH 9.6, 100mM NaCl, 5mM MgCl2, 100µl/well, 30 min or until sufficient color develops, room temp).

### Study of 3S-like microbial peptide binding to anti-3S pAb by ELISA

A rabbit anti-3S pAb was generated a BIOTEM. The ability of anti-3S pAb to bind to gp41, gp120 and gp140 proteins, 3S peptides and 3S-like microbial peptides was assessed by ELISA. 100µl of either 1mg/ml BSA, 2µg/ml of gp41, gp140 and gp120 protein or 1µg/ml of 3S or 3S-like peptides diluted in carbonate buffer, pH 9.6 (15 mM Na2CO3and 35 mM NaHCO3) were coated overnight at +4°C in duplicates on Nunc Maxisorp flat-bottom microplate (44-2404-21, Thermofisher Scientific). The unbound proteins or peptides were removed; the wells washed 2x with TBST (20mM Tris-HCI pH 7.5, 150mM NaCl, and 0.05% Tween-20) and the unreacted sites blocked by incubation (1 h, 37°C) with 300µl of 1% BSA in TBS previously heat inactivated (30 min, 56º C) and microfiltered (microfilter through a molecular sieve that has a molecular weight cut off of 3500-5000). After washing (2x with TBST), the microtiter plate bound 3S-like was incubated (1 h, 37°C) with 100 µl/well of biotinylated anti-3S pAb at 1µg/ml. After washing (2x with TBST), binding of anti-3S pAb to protein and peptides was revealed by sequential reaction with AP-Neutravidin (Pierce 31002, #HB 98661, 1 h, 37°C), three washes with TBST, and p-nitrophenyl phosphate (pNPP, Thermofisher 34045, 100mM Tris pH 9.6, 100mM NaCl, 5mM MgCl2, 100µl/well, 30 min or until sufficient color develops, room temp).

### RESULTS

### Microbial proteins bind to gC1qR to regulate the PLA2G1B inhibitory activity on CD4 T cells

As our data suggested that gp41 acts directly on PLA2G1B activity on the membrane, we developed an assay to directly address the effect of gp41 protein on PLA2G1B enzymatic activity on the membrane of CD4 T cells. Primary human CD4 T cells were labelled with tritiated arachidonic acid ([3H]AA). PLA2 enzymatic activity on the membrane releases [3H]AA in the cell supernatant. So the PLA2G1B enzymatic activity can be measured by the quantifying the radioactivity in the cell supernatant. Using this assay we demonstrated that we can study enzymatic activity of PLA2G1B on the membrane of CD4 T cells. [3H]AA is released in a PLA2G1B dose-dependent manner and is correlated with pSTAT5-NT inhibition. Moreover, gp41 directly regulates PLA2G1B activity on CD4 T cells membrane.

The gp41/3S sequence binds to the receptor for the globular head of C1q complement, gClqR (Fausther-Bovendo H et al. PLoS Pathog. 2010;6:e1000975; Pednekar L et al. Mol Immunol. 2016;74:18-26). Notably, gp41/3S peptide increases PLA2G1B activity on WT but not gClqR KO cells showing that regulation of PLA2G1B enzymatic activity by 3S is gC1qR-dependent.

gClqR was shown to bind to several microbial proteins (Ghebrehiwet B et al. Semin Immunol. 2019 Oct;45:101338). Thus, we postulated that the regulation of PLA2G1B may not be restricted to the case of HIV infection but common to other infections. These cofactors may regulate PLA2G1B enzymatic activity by targeting gC1qR. Notably, HCV core and staphylococcus aureus protein A, two proteins that are known to bind to gC1qR, also regulate PLA2G1B enzymatic activity on CD4 T cells membrane.

To identify additional gClqR binding proteins that could also act as PLA2G1B cofactors we performed an in silico screening for 3S-like motifs in protein databases. We identified 42 candidate proteins. We selected 11 proteins that contain 3S-like motif encoded by human pathogens. Biochemical binding assay to gClqR and anti-3S antibodies revealed that two peptides contain a common 3S epitope with gp41/3S and bind to gC1qR. Interestingly, one of these peptides was derived from *Porphyromonas gingivalis* that is responsible for periodontal infection and has been associated with higher risk of pancreatic cancer (Gnanasekaran J et al. Cancers (Basel) 2020 Aug 18;12(8):2331; Fan X et al. Gut 2018 Jan; 67 (1):120-127; Liu XB et al. Infect Agents and Cancer 2019 Sep 10; Torres PJ et al. PeerJ 2015 Nov 5;3:e1373; Michaud DS Carcinogenesis 2013 Oct;34(10):2193-7; Michaud DS et al. Gut 2013 Dec; 62(12):1764-70). We thus postulated that PDAC plasma could contain an inhibitory PLA2G1B activity that impaired CD4 to cells response to IL-7.

We showed that PDAC plasma inhibit CD4 T cells response to IL-7 and this inhibition is partially blocked by anti-PLA2G1B antibody. This suggests that PLA2G1B inhibition together with additional treatment could be a useful strategy to investigate to improve CD4 T-cell function and the outcome of patients with pancreatic cancer. These data also suggest that peptides derived from *Porphyromonas gingivalis* could be one of the cofactor candidates in pancreatic cancer plasma. Additional studies are required to confirm this hypothesis.

In a more general point of view our findings identified a mechanism of regulation of CD4 T cells function through the regulation of PLA2G1B activity via the gClqR by cofactors that are proteins with 3S-like motif and proteins that bind to gC1qR. This mechanism is involved in HIV-1 immunodeficiency, could play a role in pancreatic plasma and is likely to play a role in several diseases (Figure 1).

### Several proteins containing a 3S motif are encoded by human pathogens and can increase PLA2G1B activity on CD4 T-cell membrane.

Gp41 and its conserved sequence 3S are known to bind to gClqR (Fausther-Bovendo H et al. PLoS Pathog. 2010;6:e1000975; Pednekar L et al. Mol Immunol. 2016;74:18-26). We previously showed that inhibition of CD4 T-cell response due to PLA2G1B activity in VP plasma was 3S-dependent and that 3S peptide alone can increase PLA2G1B inhibitory activity on CD4 T cells (Pothlichet J et al. J Clin Invest. 2020 Jun 1;130(6):2872-2887). Thus, we postulated that gp41 could bind to gClqR to increase PLA2G1B activity on CD4 T-cell membrane. gClqR is known to bind to several proteins of microbial origins (Ghebrehiwet B et al. Semin Immunol. 2019 Oct;45:101338). One hypothesis is that the gC1qRdependent regulation of PLA2G1B activity could be a common mechanism used by pathogens to inhibit CD4 T-cell response and impair immune response.

To test this hypothesis, we tested the effect on PLA2G1B enzymatic activity on CD4 T-cell membrane of two representative microbial proteins known to bind to gClqR (Ghebrehiwet B et al. Semin Immunol. 2019 Oct;45:101338): the viral protein HCV core (Pednekar L et al. Mol Immunol. 2016;74:18-26, Kittlesen DJ et al. J Clin Invest. 2000 Nov;106(10):1239-49) and the bacterial protein A of Staphylococcus aureus (SA, Peerschke EIB et al. Infect Immun. 2006 Aug;74(8):4418-23; Nguyen T et al. Infect Immun. 2000 Apr;68(4):2061-8). Interestingly, both HCV core protein and SA protein A significantly increased PLA2G1B enzymatic activity on the membrane of CD4 T cells in a microbial protein dose-dependent manner. HCV core protein was a very potent inducer of PLA2G1B enzymatic activity. The effect of HCV core was significant with 238 nM of HCV core and PLA2G1B (at 63 nM) activity was 26-Fold higher when 595 nM of HCV core were added relatively to PLA2G1B alone at the same concentration. Although SA protein A was also a significant inducer of PLA2G1B enzymatic activity (p<0.001), the effect was lower with only 3-Fold more PLA2G1B enzymatic activity with 1190nM of SA protein A and PLA2G1B (200nM) than PLA2G1B alone. As gp41, HCV core and SA protein A are all gClqR binding proteins, these results support the hypothesis that several microbial proteins having a 3S motif are PLA2G1B cofactors that regulate PLA2G1B enzymatic activity via the binding to gClqR and thus inhibit CD4 T cell response and impair immune system.

We now propose that this cofactor effect is associated with the presence of a 3S motif in these proteins, and accordingly suggest that modified proteins with a modified 3S motif have reduced PLA2G1B cofactor effect on CD4 T cell, and thus can be used to induce a much stronger immune response when used for vaccination purpose.

### gC1qR plays a critical role in the regulation of PLA2G1B enzymatic activity by gp41.

To directly demonstrate the role of gClqR in the regulation of PLA2G1B activity by microbial proteins we searched for a CD4 T-cell line that could be labelled with [3H]AA to follow PLA2G1B activity and regulation by microbial proteins. We used 3S peptide at it was shown to bind to gClqR (Fausther-Bovendo H et al. PLoS Pathog. 2010;6:e1000975; Pednekar L et al. Mol Immunol. 2016;74:18-26). We found that gClqR is expressed in Jurkat T cells and that the gp41-derived peptide 3S can increase the PLA2G1B enzymatic activity on the membrane of Jurkat T cells. The 3S peptide effect required a pretreatment of Jurkat T cells for 4h or 21h before the addition of PLA2G1B to significantly increase PLA2G1B enzymatic activity relatively to scrambled 3S peptide-treated cells with a major effect after 21h of pretreatment p<0.0001. This last experimental condition was confirmed to be the best to study 3S effect on PLA2G1B enzymatic activity by several experiments (p<0.0001).

We thus generated gClqR clones using CRISPR/CAS9 method. We confirmed the loss of gClqR protein in gClqR KO cells relatively to WT Jurkat CD4 T cells by immunoblot revealed with two anti-gClqR mAb that bind to different part of the gClqR protein. Notably while 3S peptide significantly increased PLA2G1B enzymatic activity on WT cells relatively to scrambled 3S peptide (p<0.01) no significant change of PLA2G1B activity were observed between 3S and scrambled 3S peptides-treated cells deficient in gC1qR. Altogether these results show that gp41 can increase PLA2G1B activity through its conserved 3S region in a gC1qR-dependent manner.

### 3S-like motifs are present in proteins encoded by several human pathogens.

To identify other microbial proteins that could regulate PLA2G1B enzymatic activity on CD4 T cells and inhibit immune response, we screened protein sequences databases for similarity using Blastp with an expect threshold of 100 with a 3S amino-acid substitution matrix (AASM) based on the 3S sequence of the peptide with cofactor activity (Pothlichet J et al. J Clin Invest. 2020 Jun 1;130(6):2872-2887) and major amino-acid substitutions found in natural HIV-1 sequence variants. We identified 42 peptides with 3S-like motifs. We selected 11 peptides (Table 2) derived from 8 bacteria (Porphyromonas gingivalis (Gnanasekaran J et al. Cancers (Basel) 2020 Aug 18;12(8):2331; Fan X et al. Gut 2018 Jan; 67 (1):120-127; Liu XB et al. Infect Agents and Cancer 2019 Sep 10; Torres PJ et al. PeerJ 2015 Nov 5;3:e1373; Michaud DS Carcinogenesis 2013 Oct;34(10):2193-7; Michaud DS et al. Gut 2013 Dec; 62(12):1764-70), Proteus mirabilis, Leptospira weilii (Jayasundara D, et al. PLoS Negl Trop Dis. 2021 Mar 18;15(3):e0009272), Terrisporobacter glycolicus, Bacteroides fragilis (Cheng Y, Ling Z, Li L. Front Immunol. 2020 Nov 30;11:615056; Chung L et al. Cell Host Microbe (2018) 23(2):203-214 e205; Chao CT et al. BMC Nephrol. 2013 May 24;14:111; Kwong TNY et al. Gastroenterology 2018 Aug;155(2):383-390.e8), Aggregatibacter actinomycetemcomitans, Porphyromonas somerae, Aggregatibacter aphrophilus) and one fungi (Candida glabrata) that are involved in several human infections, have been associated with autoimmune diseases or with increased risk of cancer. Among pathogens, Porphyromonas gingivalis bacteria infection was associated with an increased risk of pancreatic cancer (Gnanasekaran J et al. Cancers (Basel) 2020 Aug 18;12(8):2331; Fan X et al. Gut 2018 Jan; 67 (1):120-127; Liu XB et al. Infect Agents and Cancer 2019 Sep 10; Torres PJ et al. PeerJ 2015 Nov 5;3:e1373; Michaud DS Carcinogenesis 2013 Oct;34(10):2193-7; Michaud DS et al. Gut 2013 Dec; 62(12):1764-70) and encoded 7 peptides with 3S-like motifs. These results showed that 3S-like motifs are not restricted to HIV or viruses but are also present in several pathogens such as bacteria and fungi.

### PLA2G1B plays a role in pancreatic ductal adenocarcinoma (PDAC) plasma inhibitory activity on pSTAT5-NT response in CD4 T cells.

Porphyromonas gingivalis infection has been associated with increased risk of pancreatic cancer (Gnanasekaran J et al. Cancers (Basel) 2020 Aug 18;12(8):2331; Fan X et al. Gut 2018 Jan; 67 (1):120-127; Liu XB et al. Infect Agents and Cancer 2019 Sep 10; Torres PJ et al. PeerJ 2015 Nov 5;3:e1373; Michaud DS Carcinogenesis 2013 Oct;34(10):2193-7; Michaud DS et al. Gut 2013 Dec; 62(12):1764-70). The severity of pancreatic cancer has been associated with impaired T-cell function and several therapeutic strategies under investigation aim to increase T cell immunity (Xiang ZJ et al. BSR 2020, Orhan A et al. EJC 2020, Brooks J et al. Cancer Res. 2018 78, 475-488, Ansari D et al. Future Oncology 2016, Chang JH et al. Medicine 2016, Saung MT et al. J Immunother Cancer. 2018 Nov 13;6(1):118, Bayne U et al. Cancer Cell 2012;21:822-35, Fogar P et al. Pancreas 2011;40:1131-7 Fukunaga A, Miyamoto M, Cho Y et al. Pancreas 2004;28:e26-31). A lower CD4 T-cell number has been reported in patients with pancreatic cancer (Chang JH et al. Medicine 2016; Helm O et al. PLoS One 2014;9:e94357; Bang S, et al. Pancreas 2006;32:29-36, Wenger FA et al. Langenbecks Arch Surg 1999;384:473-8). Thus, we postulated that the HP pg from Porphyromonas gingivalis that contains a 3S-like motif could regulate PLA2G1B activity. If so, based on our previous findings with HIV VP plasma, one could hypothesize that PDAC plasma could contain a PLA2G1B activity that will inhibit CD4 T-cell response and 3S-like peptides derived from Porphyromonas gingivalis could play the role of cofactor regulating PLA2G1B activity.

We first tested the effect of HP Pg peptide on PLA2G1B enzymatic activity in parallel with 3S peptide used as positive control. HP Pg increased PLA2G1B activity relatively to cells treated with scrambled 3S.

We then found that active PLA2G1B and proPLA2G1B concentrations were not different in PDAC and HD plasma a situation similar as HIV VP relatively to HD plasma (Pothlichet J et al. J Clin Invest. 2020 Jun 1;130(6):2872-2887).

It is of note that treatment of CD4 T cells with 3% of PDAC plasma (PDACp) impaired the pSTAT5-NT response to IL-7 (38% of inhibition) to similar level of inhibition as PLA2G1B recombinant protein (38% of inhibition). Furthermore, this effect is partially dependent on PLA2G1B activity as anti-PLA2G1B mAb significantly inhibits the activity of PDACp relatively to cells treated with plasma alone or plasma treated with control isotype (p<0.01). Anti-PLA2G1B mAb (14G9) significantly inhibits PDACp activity relatively to control isotype (p<0.01). PLA2G1B seems to be involved only in some PDAC plasma with 25 to 45 % of inhibition of PDAC plasma activity by anti-PLA2G1B mAb in 66% (4/6) of plasma tested. Altogether these data suggest that plasma from patients with pancreatic ductal adenocarcinoma can inhibit CD4 T cells response to IL-7 by a mechanism similar as we observed in HIV plasma involving the regulation of PLA2G1B activity by a cofactor that could be derived from Porphyromonas gingivalis as it encodes a 3S-like peptide that increases PLA2G1B enzymatic activity at the cell surface membrane.

### Identification of several 3S-like sequences in gp120 and gp41

The inventors identified a new mechanism of regulation of PLA2G1B by cofactors derived from pathogens. In HIV-infected patient, plasma contains gp41 fragment that drives PLA2G1B enzymatic activity on the membrane of CD4 T cells resulting in an inhibition of CD4 T cells response. This effect is dependent of conserved 3S-like sequence and gC1qR. Several pathogens encode proteins that bind to gC1qR, containing 3S-like motifs and increase PLA2G1B activity. Based on these results a position-specific scoring matrix (PSSM) was generated to predict 3S-like peptides that could be PLA2G1B cofactors.

Using this matrix we predicted 3S-like sequences that could increase PLA2G1B activity in gp120 antigen used in HIV vaccine (Figure 2). In some HIV vaccines, as for instance ALVAC HIV vCP2438, gp140 was used instead of gp120 alone. Gp140 contains gp120 and the ectodomain, i.e., the extraviral or extracellular part, of the gp41 protein. We thus extended our search for 3S-like sequences that could increase PLA2G1B activity to the gp160 of HIV strain ZM96C (idem as 96ZM651 used in vaccine ALVAC HIV vCP2438) and we found that several (four) 3S-like sequences are present in addition to the 3S sequence (Figure 2). Peptides with a score>10 are predicted to potentially increase PLA2G1B activity. One peptide is in gp120 (90-ENFNMWKNDMVDQMH-104) (SEQ ID NO: 109) and four are located in gp41 (614-PWNISWSNKSKTDIW-628 (SEQ ID NO: 117); 670-SWNNLWNWFDITKWL-684 (SEQ ID NO: 123); 672-NNLWNWFDITKWLWY-686 (SEQ ID NO: 129) and 801-GWEALKYLGSLVQYW-815(SEQ ID NO: 135)).

These 3S-like motifs in HIV antigens are likely to inhibit CD4 T cells and impair the immunization due to their inhibitory activity on CD4 T cells and consequently CD8 T cells and B-cell responses.

### Identification of 3S-like sequences present in gp120 and gp41 proteins that regulate PLA2G1B activity

The 4 3S-like sequences present in HIV strain 96ZM65, ZM96C-3S are compared with MN-3S, used as positive control, and scrambled MN-3S and scrambled ZM96C-3S peptides, used as negative controls. All peptides are compared in PLA2G1B activity assay on [3H]AA labelled Jurkat T cells or alternative assay to measure PLA2G1B activity. Critical 3S-like sequences involved in regulation of PLA2G1B activity, are identified.

The role of gClqR in this regulation is studied by investigating the effect of these peptides on PLA2G1B activity on WT vs gClqR KO Jurkat cells. We also study the binding of these WT and mutant peptides to gClqR using recombinant gClqR and to anti-3S pAb to test the hypothesis that 3S epitope are conserved.

### Identification of critical residues in 3S-like sequences present in gp120 and gp41 proteins

To identify critical residues in 3S we generate alanine substitution mutants of SWSNKS motif present in the 3S peptide from MN HIV strain as we previously demonstrated that this peptide potentiates PLA2G1B activity. All peptides, WT or variants with mutations, are compared in PLA2G1B activity assay on [3H]AA labelled Jurkat T cells or alternative assay to measure PLA2G1B activity. We have identified critical residues involved in this regulation.

To confirm the importance of these residues, mutation of homologous residues are done in 3S-like sequences present in gp120 and gp41 proteins that regulate PLA2G1B activity in previous experiments. Then WT or variants with mutations, are compared in PLA2G1B activity assay on [3H]AA labelled Jurkat T cells or alternative assay to measure PLA2G1B activity.

Thus we have identified several modifications that abrogate the increased of PLA2G1B activity by all 3S-like sequences present in gp120 and gp41.

The preferred proteins modifications are substitutions of S613 and/or W614 and/or S615 and/or N616 and/or K617 and/or S618 of 3S peptide in 3S-like sequences of gp120 and gp41 (i.e., of SWSNKS of SEQ ID NO: 73) by alanine, more preferably substitutions of W614 and/or S615 and/or N616 and/or S618. Examples of such modified 3S motifs of gp41 protein are the following:
PWNAAWSNKSLDDIW (SEQ ID NO: 45),
PWNASASNKSLDDIW (SEQ ID NO: 46),
PWNASWANKSLDDIW (SEQ ID NO: 47),
PWNASWSAKSLDDIW (SEQ ID NO: 48),
PWNASWSNASLDDIW (SEQ ID NO: 49),
PWNASWSNKALDDIW (SEQ ID NO: 50),
PWNASAANKSLDDIW (SEQ ID NO: 51),
PWNASWAAKSLDDIW (SEQ ID NO: 52),
PWNASWSAKALDDIW (SEQ ID NO: 53),
PWNASAAAKSLDDIW (SEQ ID NO: 54),
PWNASWAAKALDDIW (SEQ ID NO: 55),
PWNASAAAKALDDIW (SEQ ID NO: 56),
PWNAAASNKSLDDIW (SEQ ID NO: 57),
PWNAAWANKSLDDIW (SEQ ID NO: 58),
PWNAAWSAKSLDDIW (SEQ ID NO: 59),
PWNAAWSNASLDDIW (SEQ ID NO: 60),
PWNAAWSNKALDDIW (SEQ ID NO: 61),
PWNASWSNAALDDIW (SEQ ID NO: 62),
PWNASWSAASLDDIW (SEQ ID NO: 63),
PWNASWANASLDDIW (SEQ ID NO: 64),
PWNASASNASLDDIW (SEQ ID NO: 65),
PWNAAWANKALDDIW (SEQ ID NO: 66),
PWNAAAANKSLDDIW (SEQ ID NO: 67),
PWNAAWAAKSLDDIW (SEQ ID NO: 68),
PWNAAWSAASLDDIW (SEQ ID NO: 69),
PWNAAWSNAALDDIW (SEQ ID NO: 70),
PWNASWSAAALDDIW (SEQ ID NO: 71).

Similar alanine substitution could be done in all the 3S-like motifs identified by the inventors and indicated in the table below:

| | Protein subunit | HIV strain | Peptide sequence | ID NO |
|---|---|---|---|---|
| 3S-like seq#1 | gp120 | MN_B_P05877 | ENFNMWKNNMVEQMH | 106 |
| | | HXB2_B_P04578 | ENFNMWKNDMVEQMH | 107 |
| | | CM244_E_Q4QX31_9 | ENFNMWKNNMVEQMQ | 108 |
| | | 96ZM651_C_Q994Q6_9 | ENFNMWKNDMVDQMH | 109 |
| | | TV1_C_AAK30970.1 | ENFNMWKNDMVDQMH | 110 |
| | | 1086_C_AGV38303.1 | ENFNMWKNDMVEQMH | 111 |
| | | 92TH023_A/E_A5JP18_9 | ENFNMWKNNMVEQMQ | 112 |
| | | GNE8_B_AAV58898.1 | ENFNMWKNNMVEQMH | 113 |
| 3S sequence | gp41 | MN_B_P05877 | PWNASWSNKSLDDIW | 114 |
| | | HXB2_B_P04578 | PWNASWSNKSLEQIW | 115 |
| | | CM244_E_Q4QX31_9 | PWNSTWSNKSLEEIW | 116 |
| | | 96ZM651_C_Q994Q6_9 | PWNISWSNKSKTDIW | 117 |
| | | TV1_C_AAK30970.1 | PWNISWSNKSKTDIW | 118 |
| | | 1086_C_AGV38303.1 | PWNSSWSNKSQNEIW | 119 |
| 3S-like seq#2 | gp41 | MN_B_P05877 | KWASLWNWFDITNWL | 120 |
| | | HXB2_B_P04578 | KWASLWNWFNITNWL | 121 |
| | | CM244_E_Q4QX31_9 | KWASLWTWFDITNWL | 122 |
| | | 96ZM651_C_Q994Q6_9 | SWNNLWNWFDITKWL | 123 |
| | | TV1_C_AAK30970.1 | SWNNLWNWFDITKWL | 124 |
| | | 1086_C_AGV38303.1 | SWKNLWNWFDISKWL | 125 |
| 3S-like seq#3 | gp41 | MN_B_P05877 | ASLWNWFDITNWLWY | 126 |
| | | HXB2_B_P04578 | ASLWNWFNITNWLWY | 127 |
| | | CM244_E_Q4QX31_9 | ASLWTWFDITNWLWY | 128 |
| | | 96ZM651_C_Q994Q6_9 | NNLWNWFDITKWLWY | 129 |
| | | TV1_C_AAK30970.1 | NNLWNWFDITKWLWY | 130 |
| | | 1086_C_AGV38303.1 | KNLWNWFDISKWLWY | 131 |
| 3S-like seq#4 | gp41 | MN_B_P05877 | GWEVLKYWWNLLQYW | 132 |
| | | HXB2_B_P04578 | GWEALKYWWNLLQYW | 133 |
| | | CM244_E_Q4QX31_9 | GWEGLKYLGNLLLYW | 134 |
| | | 96ZM651_C_Q994Q6_9 | GWEALKYLGSLVQYW | 135 |
| | | TV1_C_AAK30970.1 | GWEALKYLGSLVQYW | 136 |
| | | 1086_C_AGV38303.1 | GWEILRYLGSLVQYW | 137 |

Similar alanine substitution may be done in a 3S-like motif present in gp140, for example: PWNASWSNKSLEQIW (SEQ ID NO: 161).

Similar alanine substitution could be done in 3S-like peptides present in HCV core, staphylococcus protein A and SARS_CoV-2 S1, as listed below:
Hepatitis C (core protein) 3S: P81 PWPLYGNEGCGWAGW (SEQ ID NO: 138)
Staphylococcus aureus protein A 3S: P165 KFNKEQQNAFYEILH (SEQ ID NO: 139) and P223 KFNKEQQNAFYEILH (SEQ ID NO: 140)

| **Peptide names with amino acids position in S1** | **Peptide sequence (Accession #YP_009724390.1)** | **SEQ ID NO** | **Localization in S1** |
|---|---|---|---|
| SARS-Cov-2_S1_99-113 | NIIRGWIFGTTLDSK | 141 | S1 NTD |
| SARS-Cov-2_S1_147-161 | KNNKSWMESEFRVYS | 142 | S1 NTD |
| SARS-Cov-2_S1_253-267 | DSSSGWTAGAAAYYV | 143 | S1 NTD |
| SARS-Cov-2_S1_431-445 | GCVIAWNSNNLDSKV | 144 | S1 RBD (RBM) |
| SARS-Cov-2_S1_348-362 | ASVYAWNRKRISNCV | 145 | S1 RBD |

Examples of modified 3S motifs of gp120 protein according to the invention, are the following:
ENFNAWKNNMVEQMH (SEQ ID NO: 146),
ENFNMAKNNMVEQMH (SEQ ID NO: 147),
ENFNMAANDMVEQMH (SEQ ID NO: 148),
ENFNMWANDMVEQMH (SEQ ID NO: 149),
ENFNMAANNMVEQMQ (SEQ ID NO: 150),
ENFNMWANNMVEQMQ (SEQ ID NO: 151),

An example of the modified 3S motifs of gp140 protein according to the invention, is the following:
PWNAAWANKSLEQIW (SEQ ID NO: 162).

Examples of modified 3S motifs of Hepatitis core C protein according to the invention, are the following:
PWPLAGNEGCGWAGW (SEQ ID NO: 152),
PWPLYAAEGCGWAGW (SEQ ID NO: 153).

Examples of modified 3S motifs of S.aureus A protein according to the invention, are the following:
KFNKEAANAFYEILH (SEQ ID NO:154),
KFNKEQANAFYEILH (SEQ ID NO: 155).

Examples of modified 3S motifs of SARS-CoV-2 protein according to the invention, are the following:
NIIRGAIFGTTLDSK (SEQ ID NO:156),
KNNKSAMESEFRVYS (SEQ ID NO:157),
KNNKSWAASEFRVYS (SEQ ID NO:158),
ASVYAANRKRISNCV (SEQ ID NO:159),
ASVYAAARKRISNCV (SEQ ID NO:160).

### Selection of gp41, gp120 and gp140 immunogens without PLA2G1B cofactor activity

Then we generate similar alanine substitution mutants in all 3S-like sequence in gp120 and gp41 that regulate PLA2G1B activity. WT and mutant gp120, gp41 and gp140 proteins, are tested in PLA2G1B activity assay on [3H]AA labelled Jurkat T cells, CD4 T cells or alternative assay to measure PLA2G1B activity.

### Immunogenicity assays

These mutant gp120, gp41 or gp140 proteins that do not increase PLA2G1B activity are used as immunogens vs WT sequence in mouse and macaque models. The antibody response against these proteins is followed by ELISA on coated recombinant WT and mutant proteins using anti-mouse IgG or IgM-HRP antibodies or anti-monkey IgG or IgM-HRP antibodies.

We observe an increased immune response against antigens in absence of 3S-like sequence.

### Sequence listing

SEQ ID NO: 1 (PLA2G1B) 148 aa 
Met Lys Leu Leu Val Leu Ala Val Leu Leu Thr Val Ala Ala Ala Asp
Ser Gly Ile Ser Pro Arg Ala Val Trp Gln Phe Arg Lys Met Ile Lys
Cys Val Ile Pro Gly Ser Asp Pro Phe Leu Glu Tyr Asn Asn Tyr Gly
Cys Tyr Cys Gly Leu Gly Gly Ser Gly Thr Pro Val Asp Glu Leu Asp
Lys Cys Cys Gln Thr His Asp Asn Cys Tyr Asp Gln Ala Lys Lys Leu
Asp Ser Cys Lys Phe Leu Leu Asp Asn Pro Tyr Thr His Thr Tyr Ser
Tyr Ser Cys Ser Gly Ser Ala Ile Thr Cys Ser Ser Lys Asn Lys Glu
Cys Glu Ala Phe Ile Cys Asn Cys Asp Arg Asn Ala Ala Ile Cys Phe
Ser Lys Ala Pro Tyr Asn Lys Ala His Lys Asn Leu Asp Thr Lys Lys
Tyr Cys Gln Ser
SEQ ID NO: 2 >P.gingivalis WP_088492251.1 OmpA family protein [Porphyromonas gingivalis] 
MYSGHHKIHYPFLILLVCLAFAACKSVKLKDAEKAHDRQEYTKAADMYNTLYRRTRRKQVEMKAYTAFRSGENYRA
AGRQAKALRGYLNARRYGYPDSVVLLHLAQTYQQGGNYKEAEALFRGYLEAYPKSYFAAIGLEGCLFARQQKEYPTR
YRIRRAAEWNSARGDFGPAYAPDASALYFTSSRSKDDGLDNSSITGLKPNDIYIIKRDAQGRWGRPDSVSGGINTP
WDEGVPTITPDGSTIYYTLAQQGADYDRTVQIYSAARSGEGGWSNGSLVDIMRDSLRMAAHPSMSASGDYLYFVS
NIGGSYGGKDIYRVKVSDRSYGSPENLGPDINTPGDEMFPFIDGDSTLFFASDGHAGLGGLDIFKATLDSTGRWHVV
NMGQPVNSSADDFGLAVEPKGKNKEGALPDNGVKGVFCSNRGDARGWPHLFHFELPAIYTEIQGYVMDREENLI
AGATVRIVGERGPVGQGFVTTRDDGSYKMSVQGDTRYVMLAGASGYLNQYVELKTDTAKQSETYYVDFFLASREK
AEGLQNIFYDFDKATLRPESMKSLDELIRILTDNPDIRIELGSHADRKGPDAYNLGLSDRRAKSVVDYLTSRGIAADRL
TWKGYGKSVPKTVTAKIAERHDFLKEGDVLTEEFVAPLTEEQQSVCDQLNRRTEFRVIEEELR
SEQ ID NO: 3 (GenBank reference AAC31817.1): 
AAIGALFLGFLGAAGSTMGAASVTLTVQARLLLSGIVQQQNNLLRAIESQQHMLRLTVWGIKQLQARVLAVERYLK
DQQLLGFWGCSGKLICTTTVPWNASWSNKSLDDIWNNMTWMQWEREIDNYTSLIYSLLEKSQTQQEKNEQELLE
LDKWASLWNWFDITNWLWYIKIFIMIVGGLVGLRIVFAVLSIVNRVRQGYSPLSLQTRPPVPRGPDRPEGIEEEGGE
RDRDTSGRLVHGFLAIIWVDLRSLFLLSYHHLRDLLLIAARIVELLGRRGWEVLKYWWNLLQYWSQELKSSAVSLLNA
AAIAVAEGTDRVIEVLQRAGRAILHIPTRIRQGLERALL
SEQ ID NO: 43 (GenBank: ARQ19013.1): 
MSTNPKPQRKTKRNTIRRPQDVKFPGGGQIVGGVYLLPRRGPRLGVRATRKTSERSQPRGRRQPIPKARR
PEGRTWAQPGYPWPLYGNEGMGWAGWLLSPRGSRPSWGPTDPRRRSRNLGKVIDTLTCGFADLMGYVPLV
GAPLGGAARALAHGVRALEDGVNYATGNLPGCSFSISLWXLLSCLTIPASA
SEQ ID NO: 44 (NCBI Reference Sequence: YP_498670.1): 
MKKKNIYSIRKLGVGIASVTLGTLLISGGVTPAANAAQHDEAQQNAFYQVLNMPNLNADQRNGFIQSLKD
DPSQSANVLGEAQKLNDSQAPKADAQQNNFNKDQQSAFYEILNMPNLNEAQRNGFIQSLKDDPSQSTNVL
GEAKKLNESQAPKADNNFNKEQQNAFYEILNMPNLNEEQRNGFIQSLKDDPSQSANLLSEAKKLNESQAP
KADNKFNKEQQNAFYEILHLPNLNEEQRNGFIQSLKDDPSQSANLLAEAKKLNDAQAPKADNKFNKEQQN
AFYEILHLPNLTEEQRNGFIQSLKDDPSVSKEILAEAKKLNDAQAPKEEDNNKPGKEDNNKPGKEDNNKP
GKEDNNKPGKEDNNKPGKEDGNKPGKEDNKKPGKEDGNKPGKEDNKKPGKEDGNKPGKEDGNKPGKEDGN
GVHVVKPGDTVNDIAKANGTTADKIAADNKLADKNMIKPGQELVVDKKQPANHADANKAQALPETGEENP
FIGTTVFGGLSLALGAALLAGRRREL
Modified 3S motifs of gp41: SEQ ID NO: 45 to 71:
   PWNAAWSNKSLDDIW (SEQ ID NO: 45)
   PWNASASNKSLDDIW (SEQ ID NO: 46)
   PWNASWANKSLDDIW (SEQ ID NO: 47)
   PWNASWSAKSLDDIW (SEQ ID NO: 48)
   PWNASWSNASLDDIW (SEQ ID NO: 49)
   PWNASWSNKALDDIW (SEQ ID NO: 50)
   PWNASAANKSLDDIW (SEQ ID NO: 51)
   PWNASWAAKSLDDIW (SEQ ID NO: 52)
   PWNASWSAKALDDIW (SEQ ID NO: 53)
   PWNASAAAKSLDDIW (SEQ ID NO: 54)
   PWNASWAAKALDDIW (SEQ ID NO: 55)
   PWNASAAAKALDDIW (SEQ ID NO: 56)
   PWNAAASNKSLDDIW (SEQ ID NO: 57)
   PWNAAWANKSLDDIW (SEQ ID NO: 58)
   PWNAAWSAKSLDDIW (SEQ ID NO: 59)
   PWNAAWSNASLDDIW (SEQ ID NO: 60)
   PWNAAWSNKALDDIW (SEQ ID NO: 61)
   PWNASWSNAALDDIW (SEQ ID NO: 62)
   PWNASWSAASLDDIW (SEQ ID NO: 63)
   PWNASWANASLDDIW (SEQ ID NO: 64)
   PWNASASNASLDDIW (SEQ ID NO: 65)
   PWNAAWANKALDDIW (SEQ ID NO: 66)
   PWNAAAANKSLDDIW (SEQ ID NO: 67)
   PWNAAWAAKSLDDIW (SEQ ID NO: 68)
   PWNAAWSAASLDDIW (SEQ ID NO: 69)
   PWNAAWSNAALDDIW (SEQ ID NO: 70)
   PWNASWSAAALDDIW (SEQ ID NO: 71)
SEQ ID NO: 72 (peptide 3S consensus sequence) 15aa: PWNASWSNKSLDDIW
SEQ ID NO: 73 (critical central sequence of peptide 3S) 6aa: SWSNKS
SEQ ID NO: 74 PWNASASNKSLDDIW modified 3S gp41
SEQ ID NO: 75 PWNASWANKSLDDIW modified 3S gp41
SEQ ID NO: 76 PWNASWSAKSLDDIW modified 3S gp41
SEQ ID NO: 77 PWNASWSNKALDDIW modified 3S gp41
SEQ ID NO: 78 PWNASAANKSLDDIW modified 3S gp41
SEQ ID NO: 79 PWNASWAAKSLDDIW modified 3S gp41
SEQ ID NO: 80 PWNASWSAKALDDIW modified 3S gp41
SEQ ID NO: 81 PWNASAAAKSLDDIW modified 3S gp41
SEQ ID NO: 82 PWNASWAAKALDDIW modified 3S gp41
SEQ ID NO: 83 PWNASAAAKALDDIW modified 3S gp41
SEQ ID NO: 84 (UniProtKB - A5JP18 (A5JP18_9HIV1)) gp120 
LWVTVYYGVPVWRDADTTLFCASDAKAQETEAHNVWATHACVPTDPNPQELHLENVTENFNMWKNNMVEQ
MQEDVISLWDQSLKPCVKLTPLCVTLNCTNANVTNVKNITNVPNIIGNITDEVRNCSFNMTTELRDKKQKVHALFY
KLDIVPIEDNTSSSEYRLINCNTSVIKQACPKISFDPIPIHYCTPAGYAILKCNDKNFNGTGPCKNVSSVQCTHGIKPVV
STQLLLNGSLAEEEIIIRSENLTNNAKTIIVHLNKSVEINCTRPSNNTRTSINIGPGQVFYRTGDIIGDIRKAYCEINGTKW
NEVLKKVTKKLKEHFNNKTIIFQPPSGGDLEITMHHFNCRGEFFYCNTTRLFNNTCMENETMEGCNGTIILPCKIKQII
NMWQGAGQAMYAPPISGRINCVSNITGILLTRDGGLNNTNETFRPGGGNIKDNWRSELYKYKVVQIEPLGIAPTR
AKRRVVEREKR
SEQ ID NO: 85 (UniProtKB - Q994Q6 (Q994Q6_9HIV1)) gp120 
LWVTVYYGVPVWKEAKTTLFCASDAKSYEKEVHNVWATHACVPTDPNPQEIVLGNVTENFNMWKNDMVDQM
HEDIISLWDQSLKPCVKLTPLCVTLNCTEVNVTRNVNNSVVNNTTNVNNSMNGDMKNCSFNITTELKDKKKNVYA
LFYKLDIVSLNETDDSETGNSSKYYRLINCNTSALTQACPKVSFDPIPIHYCAPAGYAILKCNNKTFNGTGPCHNVSTV
QCTHGIKPVVSTQLLLNGSLAEEGIIIRSENLTNNVKTIIVHLNRSIEIVCVRPNNNTRQSIRIGPGQTFYATGDIIGDIR
QAHCNISRTNWTKTLREVRNKLREHFPNKNITFKPSSGGDLEITTHSFNCRGEFFYCNTSGLFSINYTENNTDGTPITL
PCRIRQIINMWQEVGRAMYAPPIEGNIACKSDITGLLLVRDGGSTNDSTNNNTEIFRPAGGDMRDNWRSELYKYK
VVEIKPLGIAPTEAKRRVVEREKR
SEQ ID NO: 86 (UniProtKB - P05877 (ENV_HV1MN)) gp120 
LWVTVYYGVPVWKEATTTLFCASDAKAYDTEVHNVWATQACVPTDPNPQEVELVNVTENFNMWKNNMVEQM
HEDIISLWDQSLKPCVKLTPLCVTLNCTDLRNTTNTNNSTANNNSNSEGTIKGGEMKNCSFNITTSIRDKMQKEYAL
LYKLDIVSIDNDSTSYRLISCNTSVITQACPKISFEPIPIHYCAPAGFAILKCNDKKFSGKGSCKNVSTVQCTHGIRPVVST
QLLLNGSLAEEEVVIRSENFTDNAKTIIVHLNESVQINCTRPNYNKRKRIHIGPGRAFYTTKNIIGTIRQAHCNISRAKW
NDTLRQIVSKLKEQFKNKTIVFNQSSGGDPEIVMHSFNCGGEFFYCNTSPLFNSTWNGNNTWNNTTGSNNNITLQ
CKIKQIINMWQEVGKAMYAPPIEGQIRCSSNITGLLLTRDGGKDTDTNDTEIFRPGGGDMRDNWRSELYKYKVVTI
EPLGVAPTKAKRRVVQREKR
SEQ ID NO: 87 (UniProtKB - Q4QX31 (Q4QX31_9HIV1)) gp120 
LWVTVYYGVPVWRDADTTLFCASDAKAHETEVHNVWATHACVPTDPNPQEIDLENVTENFNMWKNNMVEQM
QEDVISLWDQSLKPCVKLTPLCVTLHCTNANLTKANLTNVNNRTNVSNIIGNITDEVRNCSFNMTTELRDKKQKVH
ALFYKLDIVPIEDNNDNSKYRLINCNTSVIKQACPKISFDPIPIHYCTPAGYAILKCNDKNFNGTGPCKNVSSVQCTHGI
KPVVSTQLLLNGSLAEEEIIIRSEDLTNNAKTIIVHLNKSVVINCTRPSNNTRTSITIGPGQVFYRTGDIIGDIRKAYCEIN
GTEWNKALKQVTEKLKEHFNNKPIIFQPPSGGDLEITMHHFNCRGEFFYCNTTRLFNNTCIANGTIEGCNGNITLPC
KIKQIINMWQGAGQAMYAPPISGTINCVSNITGILLTRDGGATNNTNNETFRPGGGNIKDNWRNELYKYKVVQIEP
LGVAPTRAKRRVVEREKR
SEQ ID NO: 88 (GenBank: AAV58898.1) gp120 
LWVTVYYGVVPVWKEATTTLFCASDAKAYDTEVHNVWATHACVPTDPNPQEIGLENVTENFNMWKNNMVEQ
MHEDIISLWDQSLKPCVKLTPLCVTLNCTDLKNATNTTSSSWGKMERGEIKNCSFNVTTSIRDKMKNEYALFYKLDV
VPIDNDNTSYRLISCNTSVITQACPKVSFEPIPIHYCAPAGFAILKCRDKKFNGTGPCTNVSTVQCTHGIRPVVSTQLLL
NGSLAEEEVVIRSANFSDNAKTIIVQLNESVEINCTRPNNNTRRSIHIGPGRAFYATGEIIGDIRQAHCNLSSTKWNNT
LKQIVTKLREHFNKTIVFNHSSGGDPEIVMHSFNCGGEFFYCNTTPLFNSTWNYTYTWNNTEGSNDTGRNITLQCRI
KQIINMWQEVGKAMYAPPIRGQIRCSSNITGLLLTRDGGNNSETEIFRPGGGDMRDNWRSELYKYKVVKIEPLGVA
PTKAKRRVVQREKR
SEQ ID NO: 89 (GenBank: AAK30970.1) gp120 
LWVTVYYGVPVWKEAKTTLFCASDAKSYEKEVHNVWATHACVPTDPNPQEIVLGNVTENFNMWKNDMVDQM
HEDIISLWDQSLKPCVKLTPLCVTLNCTEVNVTRNVNNSVVNNTTNVNNSMNGDMKNCSFNITTELKDKKKNVYA
 
LFYKLDIVSLNETDDSETGNSSKYYRLINCNTSALTQACPKVSFDPIPIHYCAPAGYAILKCNNKTFNGTGPCHNVSTV
QCTHGIKPVVSTQLLLNGSLAEEGIIIRSENLTNNVKTIIVHLNRSIEIVCVRPNNNTRQSIRIGPGQTFYATGDIIGDIR
QAHCNISRTNWTKTLREVRNKLREHFPNKNITFKPSSGGDLEITTHSFNCRGEFFYCNTSGLFSINYTENNTDGTPITL
PCRIRQIINMWQEVGRAMYAPPIEGNIACKSDITGLLLVRDGGSTNDSTNNNTEIFRPAGGDMRDNWRSELYKYK
VVEIKPLGIAPTEAKRRVVEREKR
SEQ ID NO: 90 (GenBank: AGV38303.1) gp120 
SWVTVYYGVPVWKEAKTTLFCASDAKAYEKEVHNVWATHACVPTDPNPQEMVLANVTENFNMWKNDMVEQ
MHEDIISLWDESLKPCVKLTPLCVTLNCTNVKGNESDTSEVMKNCSFKATTELKDKKHKVHALFYKLDVVPLNGNSS
SSGEYRLINCNTSAITQACPKVSFDPIPLHYCAPAGFAILKCNNKTFNGTGPCRNVSTVQCTHGIKPVVSTQLLLNGSL
AEEEIIIRSENLTNNAKTIIVHLNESVNIVCTRPNNNTRKSIRIGPGQTFYATGDIIGNIRQAHCNINESKWNNTLQKVG
EELAKHFPSKTIKFEPSSGGDLEITTHSFNCRGEFFYCNTSDLFNGTYRNGTYNHTGRSSNGTITLQCKIKQIINMWQE
VGRAIYAPPIEGEITCNSNITGLLLLRDGGQSNETNDTETFRPGGGDMRDNWRSELYKYKVVEIKPLGVAPTEAKRR
VVEREKR
SEQ ID NO: 91 (UniProtKB: P04578 (ENV_HV1H2)) gp120 
LWVTVYYGVPVWKEATTTLFCASDAKAYDTEVHNVWATHACVPTDPNPQEVVLVNVTENFNMWKNDMVEQM
HEDIISLWDQSLKPCVKLTPLCVSLKCTDLKNDTNTNSSSGRMIMEKGEIKNCSFNISTSIRGKVQKEYAFFYKLDIIPID
NDTTSYKLTSCNTSVITQACPKVSFEPIPIHYCAPAGFAILKCNNKTFNGTGPCTNVSTVQCTHGIRPVVSTQLLLNGS
LAEEEVVIRSVNFTDNAKTIIVQLNTSVEINCTRPNNNTRKRIRIQRGPGRAFVTIGKIGNMRQAHCNISRAKWNNTL
KQIASKLREQFGNNKTIIFKQSSGGDPEIVTHSFNCGGEFFYCNSTQLFNSTWFNSTWSTEGSNNTEGSDTITLPCRI
KQIINMWQKVGKAMYAPPISGQIRCSSNITGLLLTRDGGNSNNESEIFRPGGGDMRDNWRSELYKYKVVKIEPLGV
APTKAKRRVVQREKR
SEQ ID NO: 92 >gp120_A5JP18_9HIV1 Glycoprotein 120 OS=Human immunodeficiency virus 1 OX=11676 GN=env PE=4 SV=1 
MRVKETQMTWPNLWKWGTLILGLVIICSASDNLWVTVYYGVPVWRDADTTLFCASDAKAQETEAHNVWATHA
CVPTDPNPQELHLENVTENFNMWKNNMVEQMQEDVISLWDQSLKPCVKLTPLCVTLNCTNANVTNVKNITNVP
NIIGNITDEVRNCSFNMTTELRDKKQKVHALFYKLDIVPIEDNTSSSEYRLINCNTSVIKQACPKISFDPIPIHYCTPAGY
AILKCNDKNFNGTGPCKNVSSVQCTHGIKPVVSTQLLLNGSLAEEEIIIRSENLTNNAKTIIVHLNKSVEINCTRPSNNT
RTSINIGPGQVFYRTGDIIGDIRKAYCEINGTKWNEVLKKVTKKLKEHFNNKTIIFQPPSGGDLEITMHHFNCRGEFFY
CNTTRLFNNTCMENETMEGCNGTIILPCKIKQIINMWQGAGQAMYAPPISGRINCVSNITGILLTRDGGLNNTNET
FRPGGGNIKDNWRSELYKYKVVQIEPLGIAPTRAKRRVVEREKRLFIMIVGGLVGLRIVFAVLSVVNRVRQG
SEQ ID NO: 93 >gp120_AAV58898.1 truncated envelope glycoprotein, partial [Human immunodeficiency virus 1] 
VPVWKEATTTLFCASDAKAYDTEVHNVWATHACVPTDPNPQEIGLENVTENFNMWKNNMVEQMHEDIISLWD
QSLKPCVKLTPLCVTLNCTDLKNATNTTSSSWGKMERGEIKNCSFNVTTSIRDKMKNEYALFYKLDVVPIDNDNTSY
RLISCNTSVITQACPKVSFEPIPIHYCAPAGFAILKCRDKKFNGTGPCTNVSTVQCTHGIRPVVSTQLLLNGSLAEEEVV
IRSANFSDNAKTIIVQLNESVEINCTRPNNNTRRSIHIGPGRAFYATGEIIGDIRQAHCNLSSTKWNNTLKQIVTKLRE
HFNKTIVFNHSSGGDPEIVMHSFNCGGEFFYCNTTPLFNSTWNYTYTWNNTEGSNDTGRNITLQCRIKQIINMWQ
EVGKAMYAPPIRGQIRCSSNITGLLLTRDGGNNSETEIFRPGGGDMRDNWRSELYKYKVVKIEPLGVAPTKA
SEQ ID NO: 94 (UniProtKB - Q994Q6 (Q994Q6_9HIV1)) gp140 
LWVTVYYGVPVWKEAKTTLFCASDAKSYEKEVHNVWATHACVPTDPNPQEIVLGNVTENFNMWKNDMV
DQMHEDIISLWDQSLKPCVKLTPLCVTLNCTEVNVTRNVNNSVVNNTTNVNNSMNGDMKNCSFNITTE
LKDKKKNVYALFYKLDIVSLNETDDSETGNSSKYYRLINCNTSALTQACPKVSFDPIPIHYCAPAGYA
ILKCNNKTFNGTGPCHNVSTVQCTHGIKPVVSTQLLLNGSLAEEGIIIRSENLTNNVKTIIVHLNRSI
 
EIVCVRPNNNTRQSIRIGPGQTFYATGDIIGDIRQAHCNISRTNWTKTLREVRNKLREHFPNKNITFK
PSSGGDLEITTHSFNCRGEFFYCNTSGLFSINYTENNTDGTPITLPCRIRQIINMWQEVGRAMYAPPI
EGNIACKSDITGLLLVRDGGSTNDSTNNNTEIFRPAGGDMRDNWRSELYKYKVVEIKPLGIAPTEAKR
RVVEREKRAVGIGAVFLGFLGAAGSTMGAASITLTAQARQVLSGIVQQQSNLLRAIEAQQHLLQLTVW
GIKQLQTRVLAIERYLKDQQLLGLWGCSGKLICTTAVPWNISWSNKSKTDIWDNMTWMQWDREISNYT
NTIYRLLEDSQSQQEQNEKDLLALDSWNNLWNWFDITKWLWYI
SEQ ID NO: 95 (UniProtKB - Q4QX31 (Q4QX31_9HIV1)) gp140 
LWVTVYYGVPVWRDADTTLFCASDAKAHETEVHNVWATHACVPTDPNPQEIDLENVTENFNMWKNNMV
EQMQEDVISLWDQSLKPCVKLTPLCVTLHCTNANLTKANLTNVNNRTNVSNIIGNITDEVRNCSFNMT
TELRDKKQKVHALFYKLDIVPIEDNNDNSKYRLINCNTSVIKQACPKISFDPIPIHYCTPAGYAILKC
NDKNFNGTGPCKNVSSVQCTHGIKPVVSTQLLLNGSLAEEEIIIRSEDLTNNAKTIIVHLNKSVVINC
TRPSNNTRTSITIGPGQVFYRTGDIIGDIRKAYCEINGTEWNKALKQVTEKLKEHFNNKPIIFQPPSG
GDLEITMHHFNCRGEFFYCNTTRLFNNTCIANGTIEGCNGNITLPCKIKQIINMWQGAGQAMYAPPIS
GTINCVSNITGILLTRDGGATNNTNNETFRPGGGNIKDNWRNELYKYKVVQIEPLGVAPTRAKRRVVE
REKRAVGIGAMIFGFLGAAGSTMGAASITLTVQARQLLSGIVQQQSNLLRAIEAQQHLLQLTVWGIKQ
LQARVLAVERYLKDQKFLGLWGCSGKIICTTAVPWNSTWSNKSLEEIWNNMTWIEWEREISNYTNQIY
EILTKSQDQQDRNEKDLLELDKWASLWTWFDITNWLWYI
SEQ ID NO: 96 (UniProtKB - P05877 (ENV_HV1MN)) gp140 
LWVTVYYGVPVWKEATTTLFCASDAKAYDTEVHNVWATQACVPTDPNPQEVELVNVTENFNMWKNNMV
EQMHEDIISLWDQSLKPCVKLTPLCVTLNCTDLRNTTNTNNSTANNNSNSEGTIKGGEMKNCSFNITT
SIRDKMQKEYALLYKLDIVSIDNDSTSYRLISCNTSVITQACPKISFEPIPIHYCAPAGFAILKCNDK
KFSGKGSCKNVSTVQCTHGIRPVVSTQLLLNGSLAEEEVVIRSENFTDNAKTIIVHLNESVQINCTRP
NYNKRKRIHIGPGRAFYTTKNIIGTIRQAHCNISRAKWNDTLRQIVSKLKEQFKNKTIVFNQSSGGDP
EIVMHSFNCGGEFFYCNTSPLFNSTWNGNNTWNNTTGSNNNITLQCKIKQIINMWQEVGKAMYAPPIE
GQIRCSSNITGLLLTRDGGKDTDTNDTEIFRPGGGDMRDNWRSELYKYKVVTIEPLGVAPTKAKRRVV
QREKRAAIGALFLGFLGAAGSTMGAASVTLTVQARLLLSGIVQQQNNLLRAIEAQQHMLQLTVWGIKQ
LQARVLAVERYLKDQQLLGFWGCSGKLICTTTVPWNASWSNKSLDDIWNNMTWMQWEREIDNYTSLIY
SLLEKSQTQQEKNEQELLELDKWASLWNWFDITNWLWYI
SEQ ID NO: 97 (GenBank: AAK30970.1) gp140 
LWVTVYYGVPVWKEAKTTLFCASDAKSYEKEVHNVWATHACVPTDPNPQEIVLGNVTENFNMWKNDMV
DQMHEDIISLWDQSLKPCVKLTPLCVTLNCTEVNVTRNVNNSVVNNTTNVNNSMNGDMKNCSFNITTE
LKDKKKNVYALFYKLDIVSLNETDDSETGNSSKYYRLINCNTSALTQACPKVSFDPIPIHYCAPAGYA
ILKCNNKTFNGTGPCHNVSTVQCTHGIKPVVSTQLLLNGSLAEEGIIIRSENLTNNVKTIIVHLNRSI
EIVCVRPNNNTRQSIRIGPGQTFYATGDIIGDIRQAHCNISRTNWTKTLREVRNKLREHFPNKNITFK
PSSGGDLEITTHSFNCRGEFFYCNTSGLFSINYTENNTDGTPITLPCRIRQIINMWQEVGRAMYAPPI
EGNIACKSDITGLLLVRDGGSTNDSTNNNTEIFRPAGGDMRDNWRSELYKYKVVEIKPLGIAPTEAKR
RVVEREKRAVGIGAVFLGFLGAAGSTMGAASITLTAQARQVLSGIVQQQSNLLRAIEAQQHLLQLTVW
GIKQLQTRVLAIERYLKDQQLLGLWGCSGKLICTTAVPWNISWSNKSKTDIWDNMTWMQWDREISNYT
NTIYRLLEDSQSQQEQNEKDLLALDSWNNLWNWFDITKWLWYI
SEQ ID NO: 98 (GenBank: AGV38303.1) gp140 
SWVTVYYGVPVWKEAKTTLFCASDAKAYEKEVHNVWATHACVPTDPNPQEMVLANVTENFNMWKNDMV
EQMHEDIISLWDESLKPCVKLTPLCVTLNCTNVKGNESDTSEVMKNCSFKATTELKDKKHKVHALFYK
LDVVPLNGNSSSSGEYRLINCNTSAITQACPKVSFDPIPLHYCAPAGFAILKCNNKTFNGTGPCRNVS
TVQCTHGIKPVVSTQLLLNGSLAEEEIIIRSENLTNNAKTIIVHLNESVNIVCTRPNNNTRKSIRIGP
GQTFYATGDIIGNIRQAHCNINESKWNNTLQKVGEELAKHFPSKTIKFEPSSGGDLEITTHSFNCRGE
FFYCNTSDLFNGTYRNGTYNHTGRSSNGTITLQCKIKQIINMWQEVGRAIYAPPIEGEITCNSNITGL
LLLRDGGQSNETNDTETFRPGGGDMRDNWRSELYKYKVVEIKPLGVAPTEAKRRVVEREKRAVGIGAV
 
FLGFLGAAGSTMGAASMTLTVQARQLLSGIVQQQSNLLRAIEAQQHMLQLTVWGIKQLQARVLAIERY
LKDQQLLGMWGCSGKLICTTAVPWNSSWSNKSQNEIWGNMTWMQWDREINNYTNTIYRLLEDSQNQQE
KNEKDLLALDSWKNLWNWFDISKWLWYI
SEQ ID NO: 99 (UniProtKB: P04578 (ENV_HV1H2)) gp140 
LWVTVYYGVPVWKEATTTLFCASDAKAYDTEVHNVWATHACVPTDPNPQEVVLVNVTENFNMWKNDMV
EQMHEDIISLWDQSLKPCVKLTPLCVSLKCTDLKNDTNTNSSSGRMIMEKGEIKNCSFNISTSIRGKV
QKEYAFFYKLDIIPIDNDTTSYKLTSCNTSVITQACPKVSFEPIPIHYCAPAGFAILKCNNKTFNGTG
PCTNVSTVQCTHGIRPVVSTQLLLNGSLAEEEVVIRSVNFTDNAKTIIVQLNTSVEINCTRPNNNTRK
RIRIQRGPGRAFVTIGKIGNMRQAHCNISRAKWNNTLKQIASKLREQFGNNKTIIFKQSSGGDPEIVT
HSFNCGGEFFYCNSTQLFNSTWFNSTWSTEGSNNTEGSDTITLPCRIKQIINMWQKVGKAMYAPPISG
QIRCSSNITGLLLTRDGGNSNNESEIFRPGGGDMRDNWRSELYKYKVVKIEPLGVAPTKAKRRVVQRE
KRAVGIGALFLGFLGAAGSTMGAASMTLTVQARQLLSGIVQQQNNLLRAIEAQQHLLQLTVWGIKQLQ
ARILAVERYLKDQQLLGIWGCSGKLICTTAVPWNASWSNKSLEQIWNHTTWMEWDREINNYTSLIHSL
IEESQNQQEKNEQELLELDKWASLWNWFNITNWLWYI
SEQ ID NO: 100 (UniProtKB - Q994Q6 (Q994Q6_9HIV1)) gp160 
MRVREILRNWQRWWTWGILGFWMLMICNVWGNLWVTVYYGVPVWKEAKTTLFCASDAKSYEK
EVHNVWATHACVPTDPNPQEIVLGNVTENFNMWKNDMVDQMHEDIISLWDQSLKPCVKLTPL
CVTLNCTEVNVTRNVNNSVVNNTTNVNNSMNGDMKNCSFNITTELKDKKKNVYALFYKLDIV
SLNETDDSETGNSSKYYRLINCNTSALTQACPKVSFDPIPIHYCAPAGYAILKCNNKTFNGT
GPCHNVSTVQCTHGIKPVVSTQLLLNGSLAEEGIIIRSENLTNNVKTIIVHLNRSIEIVCVR
PNNNTRQSIRIGPGQTFYATGDIIGDIRQAHCNISRTNWTKTLREVRNKLREHFPNKNITFK
PSSGGDLEITTHSFNCRGEFFYCNTSGLFSINYTENNTDGTPITLPCRIRQIINMWQEVGRA
MYAPPIEGNIACKSDITGLLLVRDGGSTNDSTNNNTEIFRPAGGDMRDNWRSELYKYKVVEI
KPLGIAPTEAKRRVVEREKRAVGIGAVFLGFLGAAGSTMGAASITLTAQARQVLSGIVQQQS
NLLRAIEAQQHLLQLTVWGIKQLQTRVLAIERYLKDQQLLGLWGCSGKLICTTAVPWNISWS
NKSKTDIWDNMTWMQWDREISNYTNTIYRLLEDSQSQQEQNEKDLLALDSWNNLWNWFDITK
WLWYIKIFIMIVGGLIGLRIIFAVLSIVNRVRQGYSPLSFQTLIPNPREPDRPGRIEEEGGE
QDKERSVRLVSGFLALAWDDLRSLCLFSYHRLRDFILVTARAVELLRRSSLKGLQRGWEALK
YLGSLVQYWGLELKKSAISLLDTIAIAVAEGTDRIIELIQGICRAIRNVPRRIRQGFETALL
SEQ ID NO: 101 (UniProtKB - P05877 (ENV_HV1MN)) gp160 
MRVKGIRRNYQHWWGWGTMLLGLLMICSATEKLWVTVYYGVPVWKEATTTLFCASDAKAYDT
EVHNVWATQACVPTDPNPQEVELVNVTENFNMWKNNMVEQMHEDIISLWDQSLKPCVKLTPL
CVTLNCTDLRNTTNTNNSTANNNSNSEGTIKGGEMKNCSFNITTSIRDKMQKEYALLYKLDI
VSIDNDSTSYRLISCNTSVITQACPKISFEPIPIHYCAPAGFAILKCNDKKFSGKGSCKNVS
TVQCTHGIRPVVSTQLLLNGSLAEEEVVIRSENFTDNAKTIIVHLNESVQINCTRPNYNKRK
RIHIGPGRAFYTTKNIIGTIRQAHCNISRAKWNDTLRQIVSKLKEQFKNKTIVFNQSSGGDP
EIVMHSFNCGGEFFYCNTSPLFNSTWNGNNTWNNTTGSNNNITLQCKIKQIINMWQEVGKAM
YAPPIEGQIRCSSNITGLLLTRDGGKDTDTNDTEIFRPGGGDMRDNWRSELYKYKVVTIEPL
GVAPTKAKRRVVQREKRAAIGALFLGFLGAAGSTMGAASVTLTVQARLLLSGIVQQQNNLLR
AIEAQQHMLQLTVWGIKQLQARVLAVERYLKDQQLLGFWGCSGKLICTTTVPWNASWSNKSL
DDIWNNMTWMQWEREIDNYTSLIYSLLEKSQTQQEKNEQELLELDKWASLWNWFDITNWLWY
IKIFIMIVGGLVGLRIVFAVLSIVNRVRQGYSPLSLQTRPPVPRGPDRPEGIEEEGGERDRD
TSGRLVHGFLAIIWVDLRSLFLFSYHHRDLLLIAARIVELLGRRGWEVLKYWWNLLQYWSQE
LKSSAVSLLNATAIAVAEGTDRVIEVLQRAGRAILHIPTRIRQGLERALL
SEQ ID NO: 102 (UniProtKB - Q4QX31 (Q4QX31_9HIV1)) gp160 
MRVKETQMNWPNLWKWGTLILGLVIICSASDNLWVTVYYGVPVWRDADTTLFCASDAKAHET
EVHNVWATHACVPTDPNPQEIDLENVTENFNMWKNNMVEQMQEDVISLWDQSLKPCVKLTPL
CVTLHCTNANLTKANLTNVNNRTNVSNIIGNITDEVRNCSFNMTTELRDKKQKVHALFYKLD
IVPIEDNNDNSKYRLINCNTSVIKQACPKISFDPIPIHYCTPAGYAILKCNDKNFNGTGPCK
NVSSVQCTHGIKPVVSTQLLLNGSLAEEEIIIRSEDLTNNAKTIIVHLNKSVVINCTRPSNN
TRTSITIGPGQVFYRTGDIIGDIRKAYCEINGTEWNKALKQVTEKLKEHFNNKPIIFQPPSG
GDLEITMHHFNCRGEFFYCNTTRLFNNTCIANGTIEGCNGNITLPCKIKQIINMWQGAGQAM
YAPPISGTINCVSNITGILLTRDGGATNNTNNETFRPGGGNIKDNWRNELYKYKVVQIEPLG
VAPTRAKRRVVEREKRAVGIGAMIFGFLGAAGSTMGAASITLTVQARQLLSGIVQQQSNLLR
AIEAQQHLLQLTVWGIKQLQARVLAVERYLKDQKFLGLWGCSGKIICTTAVPWNSTWSNKSL
EEIWNNMTWIEWEREISNYTNQIYEILTKSQDQQDRNEKDLLELDKWASLWTWFDITNWLWY
IKIFIMIVGGLIGLRIIFAVLSIVNRVRQGYSPLSFQTPCHHQREPDRPERIEEEGGEQGRD
RSVRLVSGFLALAWDDLRSLCLFSYHRLRDFILIAARTVELLGRSSLKGLRRGWEGLKYLGN
LLLYWGQELKISAISLLDATAIAVAGWTDRVIEVAQGAWKAILHIPRRIRQGLERALQ
SEQ ID NO: 103 (GenBank: AAK30970.1) gp160 
MRVREILRNWQRWWTWGILGFWMLMICNVWGNLWVTVYYGVPVWKEAKTTLFCASDAKSYEK
EVHNVWATHACVPTDPNPQEIVLGNVTENFNMWKNDMVDQMHEDIISLWDQSLKPCVKLTPL
CVTLNCTEVNVTRNVNNSVVNNTTNVNNSMNGDMKNCSFNITTELKDKKKNVYALFYKLDIV
SLNETDDSETGNSSKYYRLINCNTSALTQACPKVSFDPIPIHYCAPAGYAILKCNNKTFNGT
GPCHNVSTVQCTHGIKPVVSTQLLLNGSLAEEGIIIRSENLTNNVKTIIVHLNRSIEIVCVR
PNNNTRQSIRIGPGQTFYATGDIIGDIRQAHCNISRTNWTKTLREVRNKLREHFPNKNITFK
PSSGGDLEITTHSFNCRGEFFYCNTSGLFSINYTENNTDGTPITLPCRIRQIINMWQEVGRA
MYAPPIEGNIACKSDITGLLLVRDGGSTNDSTNNNTEIFRPAGGDMRDNWRSELYKYKVVEI
KPLGIAPTEAKRRVVEREKRAVGIGAVFLGFLGAAGSTMGAASITLTAQARQVLSGIVQQQS
NLLRAIEAQQHLLQLTVWGIKQLQTRVLAIERYLKDQQLLGLWGCSGKLICTTAVPWNISWS
NKSKTDIWDNMTWMQWDREISNYTNTIYRLLEDSQSQQEQNEKDLLALDSWNNLWNWFDITK
WLWYIKIFIMIVGGLIGLRIIFAVLSIVNRVRQGYSPLSFQTLIPNPREPDRPGRIEEEGGE
QDKERSVRLVSGFLALAWDDLRSLCLFSYHRLRDFILVTARAVELLRRSSLKGLQRGWEALK
YLGSLVQYWGLELKKSAISLLDTIAIAVAEGTDRIIELIQGICRAIRNVPRRIRQGFETALL
SEQ ID NO: 104 (GenBank: AGV38303.1) gp160 
MRVRGIWKNWPQWLIWSILGFWIGNMEGSWVTVYYGVPVWKEAKTTLFCASDAKAYEKEVHN
VWATHACVPTDPNPQEMVLANVTENFNMWKNDMVEQMHEDIISLWDESLKPCVKLTPLCVTL
NCTNVKGNESDTSEVMKNCSFKATTELKDKKHKVHALFYKLDVVPLNGNSSSSGEYRLINCN
TSAITQACPKVSFDPIPLHYCAPAGFAILKCNNKTFNGTGPCRNVSTVQCTHGIKPVVSTQL
LLNGSLAEEEIIIRSENLTNNAKTIIVHLNESVNIVCTRPNNNTRKSIRIGPGQTFYATGDI
IGNIRQAHCNINESKWNNTLQKVGEELAKHFPSKTIKFEPSSGGDLEITTHSFNCRGEFFYC
NTSDLFNGTYRNGTYNHTGRSSNGTITLQCKIKQIINMWQEVGRAIYAPPIEGEITCNSNIT
GLLLLRDGGQSNETNDTETFRPGGGDMRDNWRSELYKYKVVEIKPLGVAPTEAKRRVVEREK
RAVGIGAVFLGFLGAAGSTMGAASMTLTVQARQLLSGIVQQQSNLLRAIEAQQHMLQLTVWG
IKQLQARVLAIERYLKDQQLLGMWGCSGKLICTTAVPWNSSWSNKSQNEIWGNMTWMQWDRE
INNYTNTIYRLLEDSQNQQEKNEKDLLALDSWKNLWNWFDISKWLWYIKIFIMIIGGLIGLR
IIFAVLSIVKRVRQGYSPLSFQTLTPNPRGPDRPGGIEEEGGEQDRDRSARLASGFLTLVWD
DLRSLCLFSYHLLRDFILVATRTVELLGRSSLKGLQRGWEILRYLGSLVQYWGLELKKSAIS
LIDTIAIAVAERTDRIIEIVQRICRAIRNIPRRIRQGFETALL
SEQ ID NO: 105 (UniProtKB: P04578 (ENV_HV1H2)) gp160

MRVKEKYQHLWRWGWRWGTMLLGMLMICSATEKLWVTVYYGVPVWKEATTTLFCASDAKAYD
TEVHNVWATHACVPTDPNPQEVVLVNVTENFNMWKNDMVEQMHEDIISLWDQSLKPCVKLTP
LCVSLKCTDLKNDTNTNSSSGRMIMEKGEIKNCSFNISTSIRGKVQKEYAFFYKLDIIPIDN
DTTSYKLTSCNTSVITQACPKVSFEPIPIHYCAPAGFAILKCNNKTFNGTGPCTNVSTVQCT
HGIRPVVSTQLLLNGSLAEEEVVIRSVNFTDNAKTIIVQLNTSVEINCTRPNNNTRKRIRIQ
RGPGRAFVTIGKIGNMRQAHCNISRAKWNNTLKQIASKLREQFGNNKTIIFKQSSGGDPEIV
THSFNCGGEFFYCNSTQLFNSTWFNSTWSTEGSNNTEGSDTITLPCRIKQIINMWQKVGKAM
YAPPISGQIRCSSNITGLLLTRDGGNSNNESEIFRPGGGDMRDNWRSELYKYKVVKIEPLGV
APTKAKRRVVQREKRAVGIGALFLGFLGAAGSTMGAASMTLTVQARQLLSGIVQQQNNLLRA
IEAQQHLLQLTVWGIKQLQARILAVERYLKDQQLLGIWGCSGKLICTTAVPWNASWSNKSLE
QIWNHTTWMEWDREINNYTSLIHSLIEESQNQQEKNEQELLELDKWASLWNWFNITNWLWYI
KLFIMIVGGLVGLRIVFAVLSIVNRVRQGYSPLSFQTHLPTPRGPDRPEGIEEEGGERDRDR
SIRLVNGSLALIWDDLRSLCLFSYHRLRDLLLIVTRIVELLGRRGWEALKYWWNLLQYWSQE
LKNSAVSLLNATAIAVAEGTDRVIEVVQGACRAIRHIPRRIRQGLERILL

## Claims

1. A modified HIV polypeptide, wherein said polypeptide comprises at least one modified 3S motif, and wherein said modified polypeptide has reduced PLA2G1B cofactor effect on CD4 T cells as compared to a HIV polypeptide without said modified 3S motif.

2. The modified polypeptide of claim 1, which is immunogenic.

3. The modified polypeptide of claim 2, which can cause an anti-HIV immune response in vivo.

4. The modified polypeptide of any one of claims 1 to 3, wherein the cofactor effect is reduced by at least 20%, more preferably at least 50%.

5. The modified polypeptide of any one of claims 1 to 4, wherein said polypeptide binds gClqR on CD4 T cells with less affinity than a HIV polypeptide without said modification.

6. The modified polypeptide of any one of claims 1 to 4, wherein said polypeptide has reduced immunosuppressive activity on CD4 T cells in comparison with a HIV polypeptide without said modification.

7. The modified polypeptide of any one of claims 1 to 6, wherein the 3S motif comprises 1 or more amino acid modifications selected from a substitution, addition, deletion, and/or chemical alteration, preferably at least 1, 2 or 3 amino acid modifications.

8. The modified polypeptide of claim 7, wherein said 3S motif is partially or fully deleted.

9. The modified polypeptide of claim 7, wherein said 3S motif is mutated at 1, 2, 3 or 4 amino acid residues.

10. The modified polypeptide of any one of claims 1 to 9, wherein each 3S motif present in the polypeptide is modified, in a same or different manner.

11. The modified polypeptide of any one of claims 1 to 10, wherein the polypeptide is a HIV gp140 protein or a fragment or variant thereof.

12. The modified polypeptide of any one of claims 1 to 10, wherein the polypeptide is a HIV gp120 or HIV gp160 protein or a fragment or variant thereof.

13. The modified polypeptide of any one of claims 1 to 10, wherein the polypeptide is a HIV gp41 protein or a fragment or variant thereof.

14. A nucleic acid encoding a modified polypeptide of any one of claims 1 to 3.

15. A recombinant vector comprising a nucleic acid of claim 14.

16. A host cell comprising a nucleic acid of claim 14 or a recombinant vector of claim 15.

17. A multimeric antigen comprising at least two polypeptides of any one of claims 1 to 13.

18. A polypeptide, nucleic acid, vector, cell or multimeric antigen of any one of claims 1 to 17, for use as a vaccine.

19. A polypeptide, nucleic acid, vector, cell or multimeric antigen of any one of claims 1 to 17, for use in a method of vaccinating a mammal against HIV.

20. A vaccine comprising a polypeptide, nucleic acid, vector, cell or multimeric antigen of any one of claims 1 to 17.

21. A composition comprising a polypeptide, nucleic acid, vector, cell or multimeric antigen of anyone of claims 1 to 17.

22. A method for preparing a vaccine comprising an antigen, the method comprising (i) modifying a 3S motif present in the antigen, and (ii) formulating the antigen as a vaccine.

23. A method for preparing an antigen, comprising (i) selecting an antigen of interest, and (ii) modifying a 3S motif present in the antigen so as to reduce a PLA2G1B cofactor effect of the antigen.

24. A modified polypeptide, wherein said polypeptide comprises at least one modified 3S motif, and wherein said modified polypeptide has reduced PLA2G1B cofactor effect on CD4 T cells as compared to a corresponding polypeptide without said modified 3S motif.

25. The modified polypeptide of claim 24, wherein said polypeptide is a viral or bacterial protein preferably selected from HCV core protein, SARS-CoV-2 protein, S.aureus protein A or P.gingivalis protein.
